# EUROPÄISCHE PATENTSCHRIFT

(11) **EP 3 373 812 B1**
(45) Veröffentlichungstag und Bekanntmachung des Hinweises auf die Patenterteilung: **02.10.2019**
(21) Anmeldenummer: 15801665.9
(22) Anmeldetag: 19.10.2015
(51) Int. Cl.: A61B 5/15, A61B 5/154

(54) **HANDHABUNGSVORRICHTUNG SOWIE VERFAHREN ZU DEREN HERSTELLUNG**
HANDLING DEVICE AND METHOD FOR THE PRODUCTION THEREOF
DISPOSITIF DE MANIPULATION ET PROCÉDÉ POUR LA FABRICATION DE CE DERNIER

(30) Priorität: 20.10.2014 AT 507492014
(43) Veröffentlichungstag der Anmeldung: 19.09.2018
(73) Patentinhaber: Greiner Bio-One GmbH, 4550 Kremsmünster (AT)
(72) Erfinder: EBETSBERGER, Franz, A-4550 Kremsmünster (AT); KOFLER, Georg, A-4565 Inzersdorf (AT); ÖFFERLBAUER, Florian, A-4542 Nussbach (AT)
(74) Vertreter: Ofner, Clemens
(86) Internationale Anmeldenummer: PCT/AT2015/050259
(87) Internationale Veröffentlichungsnummer: WO 2016/061601

(56) Entgegenhaltungen:
- EP-A2- 0 803 226
- WO-A1-2015/076221
- DE-A1- 2 903 167
- DE-B3-102007 031 799
- DE-U1- 20 301 575
- US-A- 3 585 984

## Beschreibung

Die Erfindung betrifft eine Handhabungsvorrichtung, insbesondere für die Medizintechnik, sowie ein Verfahren zur Herstellung einer derartigen Handhabungsvorrichtung.

Aus der EP 0 803 226 A2 ist eine gattungsgemäße Blutabnahmevorrichtung mit einem eine Kanüle aufweisenden Halter bekannt geworden. Im Halter ist die Kanüle direkt an der Frontwand gehalten und weist beidseits angeschärfte Schneidkanten auf, wobei die Kanüle in ihrem in den Halter hineinragenden Teilabschnitt von einem endseitig geschlossenen, elastischen, schlauchartigen Ventilgummi mit Luft umhüllt ist. Es wird ein sicherer Sitz des Ventilgummis in dem Halter bzw. Adapter ermöglicht, wenn der den Ventilgummi von seiner Außenseite her festlegende Abschnitt des Halters ein verformbares Klemmelement ist. Das Klemmelement kann durch einen umfangsgeschlossenen Ring oder einen Ring aus beabstandeten Teilsegmenten gebildet sein. Die Frontwand des Halters ist bis auf die Aufnahmeöffnung für die doppelendige Kanüle unterbrechungslos ausgebildet.

Die DE 29 03 167 A1 beschreibt eine Haltevorrichtung mit einer daran befestigten doppelendigen Kanüle. In der Stirnwand der Haltevorrichtung ist auf der dem Aufnahmeraum zugewendeten Seite eine zylinderförmige Ausnehmung mit radialer Distanz zur Kanüle ausgebildet, in welche Ausnehmung der offen ausgebildete Endbereich eines Schlauchventils eingesetzt und gehalten ist. Die Frontwand des Halters ist bis auf die Aufnahmeöffnung für die Kanüle unterbrechungslos ausgebildet.

Aus der US 3,585,984 A ist eine weitere Haltevorrichtung mit einer direkt an der Frontwand gehalten doppelendigen Kanüle bekannt geworden. Der distale Endbereich, in welchem die Kanüle gehalten ist, ist in Richtung auf das gegenüberliegende, offene Ende gestuft erweitern ausgebildet. In einem zylindrisch ausgebildeten Teilabschnitt ist ein Schlauchventil mit seinem kragenförmigen, umlaufenden Ansatz klemmend gehalten. Auch hier ist die Frontwand des Halters bis auf die Aufnahmeöffnung für die Kanüle unterbrechungslos ausgebildet.

Die DE 203 01 575 U1 beschreibt eine Blutabnahmevorrichtung mit einem ein Kanüle aufweisenden Halter. In einem einem Patienten zuwendbaren Frontende der Führungshülse ist ein Ringkragen mit einem Gewindeabschnitt angeordnet, in welchen die Kanüle mit einem diese aufnehmenden Kanülenhalter eingeschraubt ist. Das in den Innenraum des Halters hineinragende Ende der Kanüle ist von einem elastischen, schlauchartigen Ventilgummi umhüllt.

Der Ventilgummi weist an seinem offenen Ende einen Bund auf, der in einem Kammerraum der Führungshülse eingehängt ist und von dem das Außengewinde tragenden Kanülenhalter in Axialrichtung gegen einen in radialer Richtung auf die Längsachse vorragenden Flansch angedrückt und damit fixiert gehalten wird.

Aus der DE 10 2007 031 799 B3 ist eine Kanülenvorrichtung zur Entnahme von Rückenmarksflüssigkeiten bekannt geworden. In einer Frontwand des Halters ist eine Kanüle direkt gehalten. Der in den Innenraum des Halters ragende Teilabschnitt der Kanüle ist von einer Schutzkappe abgedeckt. Die Schutzkappe ist zu dessen Halterung an der Frontwand in einen, von der Frontwand in den Innenraum hineinragenden rohrförmigen Ansatz eingesetzt. Auch hier ist die Frontwand des Halters bis auf die Aufnahmeöffnung für die Kanüle unterbrechungslos ausgebildet.

Die DE 2 028 662 A sowie die US 3,734,080 A beschreiben eine Handhabungsvorrichtung für die Medizintechnik, welche einen Halter zur Aufnahme von Vakuumbehältern sowie eine in den Halter eingeschraubte Nadelanordnung umfasst. Die Nadelanordnung weist eine doppelendige Kanüle mit einem zwischen den beiden Kanülenenden angeordneten Halteansatz auf. Der Halteansatz trägt ein Außengewinde, mit welchem dieser in ein Gewindeloch in der Frontwand des Halters eingeschraubt werden kann. Der in den Aufnahmeraum des Halters ragende Teilabschnitt der Kanüle ist von einer elastisch verformbaren sowie durchstechbaren Absperrkappe abgedeckt. Die Absperrkappe ist dabei entweder nur an dem in den Aufnahmeraum des Halters ragende Teilabschnitt der Kanüle oder aber am Halteansatz gehalten. Dazu kann das offene Ende der Absperrkappe teilweise über den Halteansatz gestülpt sein.

Eine andere, ähnlich ausgebildete Handhabungsvorrichtung für die Blutentnahme beschreibt die DE 2 908 817 A. Diese umfasst einen hohlzylindrischen Halter, in dessen einem ersten Ende eine Nadelanordnung mit einer doppelendigen hohlen Nadel angeordnet ist und dessen anderes zweites Ende offen und zur Aufnahme eines Blutprobenröhrchens ausgebildet ist. An der doppelendigen hohlen Nadel ist ein eigener Nadelhalter mit einem Außengewinde befestigt. Der Halter weist zur Aufnahme und Halterung des Nadelhalters an seinem ersten Ende eine Gewindebohrung auf. Der sich in den Innenraum des Halters hinein erstreckende Teil der hohlen Nadel ist von einer Schutzhülle aus leicht durchstechbaren, weichem Material umgeben. Die Schutzhülle ist am Nadelhalter der einschraubbaren Nadelanordnung gehalten.

Die WO 95/16395 A1 sowie die US 5,897,508 A beschreiben ebenfalls Haltevorrichtungen für Blutprobenentnahmeröhrchen. Im hohlzylindrisch ausgebildeten Halter ist an dessen Frontwand ein eigener Nadelhalter über einen tellerartigen Trägerteil daran befestigt. Vom Trägerteil ragt ein rohrförmig ausgebildeter Nadelträger vor, an welchem über eine Haltenase das elastisch verformbar ausgebildete Schlauchventil gehalten ist. Das Schlauchventil deckt die im Nadelträger gehaltene Kanüle ab.

Nachteilig bei all diesen Haltevorrichtungen ist, dass die Schutzhülle bzw. das Schlauchventil stets unter Zwischenschaltung eines weiteren Bauteils an dem hohlzylindrisch ausgebildeten Halter gehalten ist.

Der vorliegenden Erfindung liegt die Aufgabe zugrunde, eine Handhabungsvorrichtung sowie ein Verfahren zu deren Herstellung zu schaffen, bei welcher bzw. bei welchem im Zuge der Herstellung die Prozesssicherheit erhöht und mögliche Stillstandszeiten herabgesetzt oder überhaupt vermieden werden können. Darüber hinaus soll bei derartigen in hohen Stückzahlen gefertigten Einwegprodukten die Herstellung vereinfacht werden.

Diese Aufgabe der Erfindung wird dadurch gelöst, dass die Kanüle direkt mit der Frontwand des Hauptkörperteils verbunden ist, oder dass die Kanüle an der Frontwand als integraler Bestandteil des Hauptkörperteils ausgebildet ist und das mindestens eine Halteelement eine am Haltearm angeordnete sowie in Richtung auf die Längsachse vorragende Haltenase aufweist und dass in der Frontwand in einem in Axialrichtung gesehen liegenden Projektionsabschnitt des zumindest einen Halteelements neben diesem in radialer Richtung auf die Längsachse hin, ein die Frontwand durchsetzender Durchbruch ausgebildet ist, und ein Querschnitt des Durchbruchs in Axialrichtung zumindest einer Projektionsfläche der Haltenase entspricht.

Der dadurch erzielte überraschende Vorteil liegt darin, dass damit eine gerichtete Anlage des Halteelements an der Außenseite der Hüllenwand erreicht werden kann. Durch das Vorsehen des Haltearms kann so darüber hinaus je nach dessen Ausbildung die damit aufgebaute Rückhaltewirkung erreicht werden. Diese kann formschlüssig und/oder kraftschlüssig auf Reibungswirkung beruhend ausgebildet sein. Durch das Vorsehen des zumindest einen Durchbruchs kann so die Ausformung des mindestens einen Halteelements einfacher formtechnisch erfolgen. Damit kann auf komplizierte Ausbildungen im Bereich des Spritzgusswerkzeuges verzichtet werden, da die Öffnungsbewegung durch eine einfache Auf- und Zubewegung zur Bildung des Formhohlraums durchgeführt werden kann. So kann das Spritzgusswerkzeug einfacher gestaltet und auch leistungsstärker aufgrund geringerer Verstellbewegungen betrieben werden.

Durch die direkte Verbindung der Kanüle mit der Frontwand kann so auf zusätzliche an der Kanüle ansonsten angeordnete Verbindungsteile verzichtet werden. Eine zusätzliche Vereinfachung des Montageprozesses kann auch dadurch erreicht werden, wenn die Kanüle in eine im Bereich der Frontwand ausgebildete Aufnahmeöffnung eingepresst ist. Dadurch können mehrere Prozessschritte für den Verbindungsvorgang entfallen. Damit kann das Vorbehandeln der zu verbindenden Bauteile, das Dosieren des Klebemittels sowie das nachfolgende Aushärten eingespart werden. So kann die Prozesssicherheit für den Herstellvorgang wesentlich erhöht werden. Weiters kann damit, wenn die Kanüle an der Frontwand als integraler Bestandteil des Hauptkörperteils ausgebildet ist, auf das eigene Ausbilden der Kanüle sowie dessen Verbindungsvorgang mit dem Hauptkörperteil verzichtet werden. So kann in einem Arbeitsgang, insbesondere in einem Spritzgussvorgang, in einem einteiligen Bauteil bis auf die Anordnung der Hülle als Schlauchventil ein überwiegender Teil der Handhabungsvorrichtung hergestellt werden. Durch den Wegfall dieser Prozessschritte können die Anlagen zum Fügen des Hauptkörperteils mit der Kanüle wegfallen. Durch den Wegfall der zusätzlichen Anlagenteile können damit aber auch Anschaffungskosten sowie Betriebskosten eingespart werden.

Für die Halterung und Befestigung der Hülle muss so kein zusätzlicher Bauteil vorgesehen werden. Dadurch kann die Herstellung im Spritzgussprozess ohne zusätzliche Entformschritte mittels eines einfacher ausgebildeten Spritzgusswerkzeuges erfolgen, da in diesem Bereich Hinterschneidungen vermieden werden. Durch das Anordnen bzw. Vorsehen von zumindest einem Halteelement an der Frontwand kann so die relative Lagefixierung der Hülle im Bereich der Frontwand erreicht werden. Durch das Anliegen von dem mindestens einen Halteelement an der Außenseite der Hülle kann so entweder eine kraftschlüssige und/oder formschlüssige Verbindung zwischen der Hülle und der Frontwand geschaffen werden. Damit kann eine axiale Lagefixierung der Hülle erzielt und ein unbeabsichtigtes Abziehen der Hülle von der Frontwand verhindert werden.

Eine weitere mögliche Ausführungsform hat die Merkmale, dass die Haltevorrichtung einen in etwa zylindrisch oder kegelstumpfförmig ausgebildeten Zentrieransatz aufweist, welcher integraler Bestandteil der Frontwand ist, und der Zentrieransatz in das offene distale Hüllenende der Hülle hineinragt. Da der Zentrieransatz für die Aufnahme der Hülle direkt am hohl ausgebildeten Hauptkörperteil, insbesondere dessen Frontwand, einstückig daran angeordnet sowie ausgebildet ist, kann die Hülle einfach darauf aufgesetzt bzw. übergestülpt werden. Darüber hinaus kann der Zentrieransatz auch noch als Gegenhalter bzw. Gegenanschlag für das zumindest eine Halteelement dienen.

Eine weitere bevorzugte Ausführungsform ist dadurch gekennzeichnet, dass das zumindest eine Halteelement in einer radialen Distanz unter Ausbildung eines Spaltes vom Zentrieransatz angeordnet ist. Damit kann die Formgebung in einem einfachen Spritzgussvorgang erfolgen und auch die Ausformung ohne zusätzliche Schieberbewegungen rasch und einfach durchgeführt werden.

Weiters ist es vorteilhaft, wenn die radiale Distanz zwischen dem zumindest einen Halteelement und dem Zentrieransatz bei anliegendem Halteelement an der Hülle einen Wert aufweist, welcher aus einem Wertebereich stammt, dessen untere Grenze 5% und dessen obere Grenze 95% bezüglich der unverformten Wandstärke der Hülle beträgt. So kann durch die Wahl der Spaltweite eine Verformung der Hüllenwand im Kontaktbereich mit dem Halteelement erzielt und damit verbunden eine Klemmwirkung aufgebaut werden. Damit kann einerseits eine Rückhaltekraft zwischen der Innenfläche der Hülle und dem Zentrieransatz und andererseits durch die Umformung der Hüllenwand ein mechanisch wirkender Rückhalteansatz zwischen dieser und dem Halteelement geschaffen werden.

Eine weitere mögliche Ausführungsform hat die Merkmale, dass zumindest die Haltenase des mindestens einen Halteelements im Kontaktbereich an einer Hüllenwand der Hülle anliegt. Damit kann je nach ausgeübter Druckkraft der Haltenase die Klemmwirkung für die Hülle zwischen der Haltenase und dem Zentrieransatz festgelegt werden.

Eine weitere Ausbildung sieht vor, dass die Haltenase in Axialrichtung von der Frontwand distanziert angeordnet ist. Damit kann ein gewisser Überlappungsbereich bzw. Überstand des Kontaktbereichs von der Frontwand erreicht werden. Weiters kann damit aufgrund des Überragens ein gewisser Sicherheitsbereich geschaffen werden, innerhalb welchem die Hülle am Zentrieransatz in Axialrichtung aufgesetzt werden muss. Auch bei einem geringen Abziehen oder einem nicht ganz vollständigen Aufsetzen der Hülle auf den Zentrieransatz kann dabei trotzdem die Halterung der Hülle in Axialrichtung am Zentrieransatz durch das zumindest eine Halteelement erreicht werden.

Eine mögliche Weiterbildung dazu sieht vor, dass der die Frontwand durchsetzende Durchbruch zwischen dem Haltearm des zumindest einen Halteelements und dem Zentrieransatz ausgebildet ist. Damit kann auch bei Vorhandensein des Zentrieransatzes auf komplizierte Ausbildungen des Spritzgusswerkzeuges im Bereich der Frontwand verzichtet werden.

Eine weitere bevorzugte Ausführungsform ist dadurch gekennzeichnet, dass die Hülle im Bereich ihres offenen distalen Hüllenendes einen in radialer Richtung über die Hülle vorragenden Kragen aufweist. Durch den die Hülle radial überragenden Kragen kann so ein zusätzliches Rückhalteelement an der Hülle geschaffen werden. Weiters kann damit aber auch die Eigensteifigkeit der Hülle in dessen offenen, distalen Hüllendes erhöht werden.

Weiters ist es vorteilhaft, wenn die Haltenase des mindestens einen Halteelements den Kragen der Hülle an seiner dem offen ausgebildeten, proximalen Ende des Hauptkörperteils zugewendeten Seite hintergreift. So kann durch das Hintergreifen des Kragens durch die Haltenase zusätzlich ein formschlüssiges Rückhalteelement im Bereich der Haltevorrichtung für die Hülle ausgebildet werden. Damit kann die Rückhaltewirkung der Haltevorrichtung noch zusätzlich verbessert werden.

Eine andere Ausführungsform zeichnet sich dadurch aus, dass das mindestens eine Halteelement verstellbar, insbesondere verschwenkbar, an der Frontwand gehalten ist. Durch die verschwenkbare Anordnung und Halterung des mindestens einen Halteelements kann so die Aufsetzbewegung der Hülle auf den Zentrieransatz erleichtert werden. Darüber hinaus kann so aber auch eine selbsttätige Halterung der Hülle am Zentrieransatz durch die selbsttätige Rückstellung des mindestens einen Halteelements in dessen Haltestellung erreicht werden. Damit kann die Montage der Hülle am hohlzylindrisch ausgebildeten Hauptkörperteil vereinfacht werden.

Eine weitere mögliche Ausführungsform hat die Merkmale, dass die direkt mit der Frontwand des Hauptkörperteils verbundene Kanüle in eine im Bereich der Frontwand ausgebildete Aufnahmeöffnung eingepresst ist, wodurch eine zusätzliche Vereinfachung des Montageprozesses erreicht werden kann. Dadurch können mehrere Prozessschritte für den Verbindungsvorgang entfallen. Damit kann das Vorbehandeln der zu verbindenden Bauteile, das Dosieren des Klebemittels sowie das nachfolgende Aushärten eingespart werden. So kann die Prozesssicherheit für den Herstellvorgang wesentlich erhöht werden.

Eine weitere Ausbildung sieht vor, dass sowohl die Aufnahmeöffnung für die Kanüle als auch der Zentrieransatz für die Hülle jeweils in der Längsachse liegend angeordnet sind. Damit kann eine zentrische Anordnung der Kanüle sowie der Hülle innerhalb des Aufnahmeraums des hohlzylindrisch ausgebildeten Hauptkörperteils erzielt werden.

Eine andere Ausführungsform zeichnet sich dadurch aus, dass die Kanüle am Zentrieransatz als integraler Bestandteil des Hauptkörperteils ausgebildet ist. Damit kann auf das eigene Ausbilden der Kanüle sowie dessen Verbindungsvorgang mit dem Hauptkörperteil verzichtet werden. So kann in einem Arbeitsgang, insbesondere in einem Spritzgussvorgang, in einem einteiligen Bauteil bis auf die Anordnung der Hülle als Schlauchventil ein überwiegender Teil der Handhabungsvorrichtung hergestellt werden. Durch den Wegfall dieser Prozessschritte können die Anlagen zum Fügen des Hauptkörperteils mit der Kanüle wegfallen. Durch den Wegfall der zusätzlichen Anlagenteile können damit aber auch Anschaffungskosten sowie Betriebskosten eingespart werden.

Eine weitere bevorzugte Ausführungsform ist dadurch gekennzeichnet, dass an der Frontwand an der vom Aufnahmeraum abgewendeten Seite ein über die Frontwand in Axialrichtung vorragendes Anschlussstück ausgebildet ist. Damit wird die Möglichkeit geschaffen, die Handhabungsvorrichtung mit den unterschiedlichsten zusätzlichen Bauteilen kuppeln und/oder verbinden zu können. Je nach Ausbildung des Anschlussstücks kann so eine hohe Vielfalt an Verbindungs- und/oder Kupplungsmöglichkeiten geschaffen werden.

Eine andere Ausführungsform zeichnet sich dadurch aus, dass die Kanüle mit ihrem distalen Kanülenende zumindest teilweise in das Anschlussstück hineinragt. Bedingt durch dieses Hineinragen kann eine bessere Halterung und damit größere Klemmlänge der Kanüle im Hauptkörperteil erzielt werden.

Eine weitere mögliche Ausführungsform hat die Merkmale, dass die Kanüle mit ihrem distalen Kanülenende das Anschlussstück in Axialrichtung überragt und an ihrem distalen Kanülenende ein Einstichende zum Einstechen in einen Körperteil aufweist. So wird die Möglichkeit geschaffen bei Ausbildung einer doppelendigen Kanüle die Handhabungsvorrichtung direkt ohne Anordnung von zusätzlichen Bauteilen für das Abnehmen bzw. Aufsammeln von Körperflüssigkeiten, insbesondere Blut, einsetzen zu können.

Eine weitere Ausbildung sieht vor, dass am Anschlussstück ein Adapter positioniert gehalten ist und zwischen dem Anschlussstück und dem Adapter zumindest ein Dichtabschnitt ausgebildet ist. Durch die zusätzliche Möglichkeit, am Anschlussstück auch noch ein Adapterstück anordnen und dort auch positioniert halten zu können, werden die universellen Einsatzmöglichkeiten der Handhabungsvorrichtung noch weiter erhöht. Aufgrund der inneren gegenseitigen Abdichtung wird ein Ansaugen von Falsch- bzw. Fremdluft während des Abnahmevorgangs mit einem evakuierten Abnahmebehälter verhindert. Darüber hinaus kann aber auch einer der Dichtabschnitte auch einen Zentrierabschnitt ausbilden.

Die Aufgabe der Erfindung kann aber unabhängig davon auch durch ein Verfahren zur Herstellung einer Handhabungsvorrichtung dadurch gelöst werden, wenn dabei folgende Schritte durchgeführt werden:
- Ausbilden einer schlauchförmigen, elastisch verformbaren sowie durchstechbaren Hülle mit einem offenen distalen Hüllenende sowie einem verschlossenen proximalen Hüllenende;
- Ausbilden eines hohlen Hauptkörperteils mit einem mit einer Frontwand zumindest teilweise verschlossenen distalen Ende und einem offenen proximalen Ende, bei dem vom Hauptkörperteil sowie von der Frontwand ein Aufnahmeraum definiert wird, und das proximale Ende zur Aufnahme zumindest eines Teilabschnitts eines Aufnahmebehälters im Aufnahmeraum dient, wobei sich zwischen dem distalen Ende und dem proximalen Ende eine Längsachse (7) erstreckt;
- Ausbilden einer Haltevorrichtung für die Hülle direkt im Bereich der Frontwand an ihrer dem Aufnahmeraum zugewendeten Seite, wobei die Haltevorrichtung durch zumindest ein an der Frontwand ausgebildetes oder an der Frontwand angeordnetes Halteelement gebildet wird, wobei das zumindest eine Halteelement durch einen von der Frontwand vorragenden Haltearm gebildet wird;
- Ausbilden einer Haltenase am Haltearm des mindestens einen Halteelements, wobei die Haltenase in Richtung auf die Längsachse vorragend ausgebildet wird;
- Ausbilden eines die Frontwand durchsetzenden Durchbruchs, wobei der Durchbruch in einem in Axialrichtung gesehen liegenden Projektionsabschnitt des zumindest einen Halteelements neben diesem in radialer Richtung auf die Längsachse hin ausgebildet wird und ein Querschnitt des Durchbruchs in Axialrichtung zumindest mit einer der Haltenase entsprechenden Projektionsfläche ausgebildet wird;
- Anordnen und Verbinden einer als Kanüle ausgebildeten Nadelanordnung direkt mit der Frontwand des Hauptkörperteils oder Ausbilden einer als Kanüle ausgebildeten Nadelanordnung an der Frontwand als integraler Bestandteil des Hauptkörperteils, sodass die Kanüle ausgehend von der Frontwand mit ihrem proximalen Kanülenende in den Aufnahmeraum hineinragt;
- Verbringen der Hülle mit ihrem offenen distalen Hüllenende in den Bereich des zumindest einen an der Frontwand ausgebildeten oder an der Frontwand angeordneten Halteelements und dabei Abdecken der in den Aufnahmeraum hineinragenden Kanüle;
- Herstellen eines Kontaktbereichs zwischen dem Halteelement und der Hülle, in welchem das Halteelement außenseitig an der Hülle zur Anlage gebracht wird.

Vorteilhaft ist bei den hier gewählten Verfahrensschritten, dass damit eine gerichtete Anlage des Halteelements an der Außenseite der Hüllenwand erreicht werden kann. Durch das Vorsehen des Haltearms kann so darüber hinaus je nach dessen Ausbildung die damit aufgebaute Rückhaltewirkung erreicht werden. Diese kann formschlüssig und/oder kraftschlüssig auf Reibungswirkung beruhend ausgebildet werden. Durch das Vorsehen des zumindest einen Durchbruchs kann so die Ausformung des mindestens einen Halteelements einfacher formtechnisch erfolgen. Damit kann auf komplizierte Ausbildungen im Bereich des Spritzgusswerkzeuges verzichtet werden, da die Öffnungsbewegung durch eine einfache Auf- und Zubewegung zur Bildung des Formhohlraums durchgeführt werden kann. So kann das Spritz gusswerkzeug einfacher gestaltet und auch leistungsstärker aufgrund geringerer Verstellbewegungen betrieben werden.

Für die Halterung und Befestigung der Hülle muss so kein zusätzlicher Bauteil vorgesehen werden. Dadurch kann die Herstellung im Spritzgussprozess ohne zusätzliche Entformschritte mittels eines einfacher ausgebildeten Spritzgusswerkzeuges erfolgen, da in diesem Bereich Hinterschneidungen vermieden werden. Durch das Anordnen bzw. Vorsehen von zumindest einem Halteelement an der Frontwand kann so die relative Lagefixierung der Hülle im Bereich der Frontwand erreicht werden. Durch das Anliegen von dem mindestens einen Halteelement an der Außenseite der Hülle kann so entweder eine kraftschlüssige und/oder formschlüssige Verbindung zwischen der Hülle und der Frontwand geschaffen werden. Damit kann eine axiale Lagefixierung der Hülle erzielt und ein unbeabsichtigtes Abziehen der Hülle von der Frontwand verhindert werden.

Vorteilhaft ist auch eine Verfahrensvariante, bei der an der Frontwand als weiterer Bauteil der Haltevorrichtung ein in etwa zylindrisch oder kegelstumpfförmig ausgebildeten Zentrieransatz als integraler Bestandteil der Frontwand ausgebildet wird, welcher Zentrieransatz in das offene distale Hüllenende der Hülle hineinragend ausgebildet wird. Da der Zentrieransatz für die Aufnahme der Hülle direkt am hohl ausgebildeten Hauptkörperteil, insbesondere dessen Frontwand, einstückig daran angeordnet sowie ausgebildet ist, kann die Hülle einfach darauf aufgesetzt bzw. übergestülpt werden. Darüber hinaus kann der Zentrieransatz auch noch als Gegenhalter bzw. Gegenanschlag für das zumindest eine Halteelement dienen.

Eine weitere vorteilhafte Vorgehensweise sieht vor, dass das zumindest eine Halteelement in einer radialen Distanz unter Ausbildung eines Spaltes vom Zentrieransatz angeordnet wird. Damit kann die Formgebung in einem einfachen Spritzgussvorgang erfolgen und auch die Ausformung ohne zusätzliche Schieberbewegungen rasch und einfach durchgeführt werden.

Vorteilhaft kann auch ein Vorgehen sein, bei dem der die Frontwand durchsetzende Durchbruch zwischen dem Haltearm des zumindest einen Halteelements und dem Zentrieransatz ausgebildet wird. Damit kann auch bei Vorhandensein des Zentrieransatzes auf komplizierte Ausbildungen des Spritzgusswerkzeuges im Bereich der Frontwand verzichtet werden.

Eine andere Vorgehensweise ist dadurch gekennzeichnet, dass die Hülle im Bereich ihres offenen distalen Hüllenendes mit einen in radialer Richtung über die Hülle vorragenden Kragen ausgebildet wird. Durch den die Hülle radial überragenden Kragen kann so ein zusätzliches Rückhalteelement an der Hülle geschaffen werden. Weiters kann damit aber auch die Eigensteifigkeit der Hülle in dessen offenen, distalen Hüllendes erhöht werden.

Vorteilhaft ist auch eine Verfahrensvariante, bei der zwischen dem zumindest einen Halteelement und dem Zentrieransatz ausgebildete Spalt vor dem Aufsetzen der Hülle auf den Zentrieransatz mit einer bezüglich der Wandstärke der Hülle dazu größeren Distanz ausgebildet wird und nach dem Verbringen der Hülle in den Spalt das mindestens eine Halteelement hin in Richtung auf die Längsachse verlagert und damit außenseitig an der Hülle im Kontaktbereich zur Anlage gebracht wird. Damit kann die Aufsetzbewegung der Hülle auf den Zentrieransatz vereinfacht werden. Weiters kann damit aber auch die notwendige Anlage des mindestens einen Halteelements und/oder das Herstellen der Rückhaltewirkung der Halteelemente individuell an die Ausbildung der Hülle angepasst werden. Das Verlagern des mindestens einen Halteelements zum Aufbau der Rückhaltewirkung der Hülle kann durch einen Umformvorgang des zumindest einen Halteelements durchgeführt werden. So wäre es möglich, durch entsprechende Erhöhung der Temperatur zumindest des den das Halteelement bildenden Werkstoff soweit zu erweichen, dass dieser ohne Bruch zur Anlage an der Außenseite der Hülle umgeformt werden kann.

Zum besseren Verständnis der Erfindung wird diese anhand der nachfolgenden Figuren näher erläutert.

Es zeigen jeweils in stark vereinfachter, schematischer Darstellung:
- Fig. 1: eine erste mögliche Ausbildung der Handhabungsvorrichtung, im Axialschnitt;
- Fig. 2: eine zweite Ausführungsform der Handhabungsvorrichtung, in einem axialen Teilschnitt sowie schaubildlicher Darstellung;
- Fig. 3: die Handhabungsvorrichtung nach Fig. 2, im Axialschnitt;
- Fig. 4: eine dritte mögliche Ausbildung der Handhabungsvorrichtung, im Axialschnitt;
- Fig. 5: eine weitere Handhabungsvorrichtung mit einem im Bereich von dessen Anschlussstück angeordneten Adapter, im Axialschnitt;
- Fig. 6: eine weitere andere mögliche Ausbildung der Handhabungsvorrichtung, im Axialschnitt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen. Der Begriff "insbesondere" wird nachfolgend so verstanden, dass es sich dabei um eine mögliche speziellere Ausbildung oder nähere Spezifizierung eines Gegenstandes handeln kann, aber nicht unbedingt eine zwingende, bevorzugte Ausführungsform desselben darstellen muss.

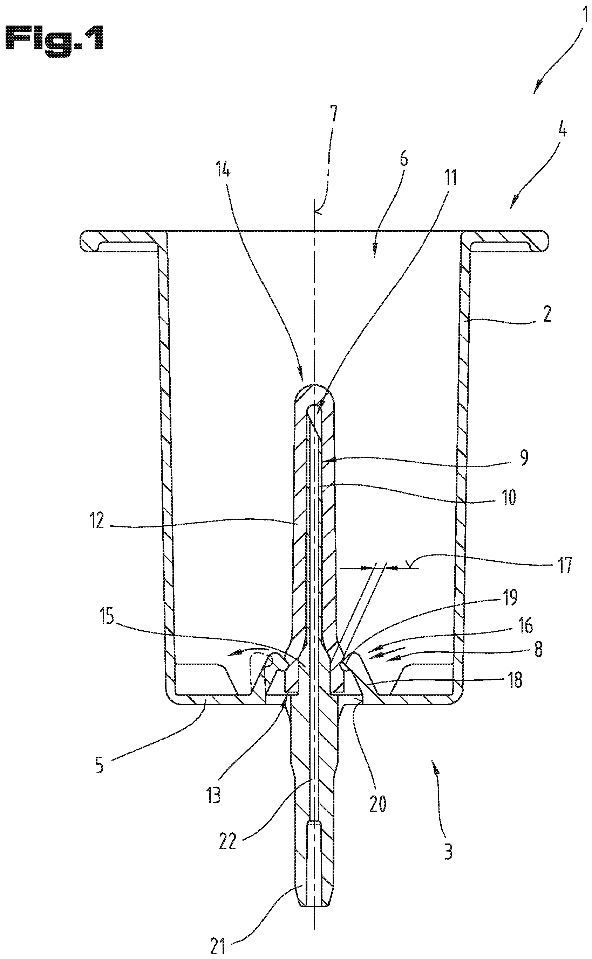

In der Fig. 1 ist ein erstes Ausführungsbeispiel einer Handhabungsvorrichtung 1 für eine medizinische Baugruppe, insbesondere zur Verwendung in der Medizintechnik gezeigt. So kann die Handhabungsvorrichtung 1 für die Abnahme oder das Aufsammeln von Körperflüssigkeiten, insbesondere Blut, Urin und/oder die Abgabe von Medikamenten in den Körper verwendet werden. Dabei ist die Handhabungsvorrichtung 1 derart ausgebildet, dass diese an einem Endbereich mit den unterschiedlichsten medizinischen Hilfsmitteln verbunden insbesondere gekuppelt werden kann und an dem davon abgewendeten Endbereich zur Aufnahme zumindest eines Teils eines hier nicht näher dargestellten Aufnahmebehälters ausgebildet ist, welcher Aufnahmebehälter auch als Blutentnahmeröhrchen oder Blutsammelröhrchen gezeichnet werden kann. Bei derartigen Aufnahmebehältern kann auch deren Innenraum auf einen bezüglich des Umgebungsdrucks dazu reduzierten Druck abgesenkt sein. Dies kann somit auch als Vakuumröhrchen bezeichnet werden.

Die Handhabungsvorrichtung 1 umfasst ihrerseits bei diesem Ausführungsbeispiel einen hohl ausgebildeten Hauptkörperteil 2 der ein distales Ende 3 und ein proximales Ende 4 aufweist. Dabei kann der Hauptkörperteil 2 in etwa rohrförmig ausgebildet sein und auch als Halter mit einer Halterwand bezeichnet werden. Weiters können die unterschiedlichsten Querschnittsabmessungen sowie Querschnittformen Anwendung finden, wobei dies in Abhängigkeit von den Abmessungen des aufzunehmenden Aufnahmebehälters zu wählen ist. Es ist auch eine leichte, konische Form mit oder ohne Abstufungen möglich.

Im vorliegenden Ausführungsbeispiel ist das distale Ende 3 zumindest teilweise mit einer Frontwand 5 verschlossen. Der Hauptkörperteil 2 mit seiner Behälterwand sowie die Frontwand 5 umgrenzen bzw. definieren einen Aufnahmeraum 6. Um den zuvor beschriebenen Teilabschnitt des Aufnahmebehälters im Aufnahmeraum 6 aufnehmen zu können, ist das proximale Ende 4 in seinen Abmessungen entsprechend ausgebildet. Weiters erstreckt sich zwischen dem distalen Ende 3 und dem proximalen Ende 4 eine Längsachse 7. Im Bereich des offenen proximalen Endes 4 ist zumeist eine nicht näher bezeichnete Handhabe in Form eines flanschförmigen Ansatzes angeordnet bzw. ausgebildet.

Weiters ist hier noch gezeigt, dass im Bereich der Frontwand 5 an ihrer dem Aufnahmeraum 6 zugewendeten Seite eine Haltevorrichtung 8 für eine Schutzhülle angeordnet ist, welche später noch beschrieben wird.

Die Handhabungsvorrichtung 1 umfasst weiters eine Nadelanordnung 9, welche als Kanüle 10 ausgebildet ist. Unter Kanüle 10 wird im vorliegenden Ausführungsbeispiel eine hohl ausgebildete Nadel verstanden, welche in ihrem Inneren einen bevorzugt durchgängigen Durchströmkanal definiert bzw. ausbildet.

So ragt bei diesem Ausführungsbeispiel die Kanüle 10 ausgehend von der Frontwand 5 mit ihrem proximalen Kanülenende 11 in den Aufnahmeraum 6 des Hauptkörperteils 2 hinein. Weiters ist die Kanüle 10 mit dem Hauptkörperteil 2, insbesondere dessen Frontwand 5, gekoppelt.

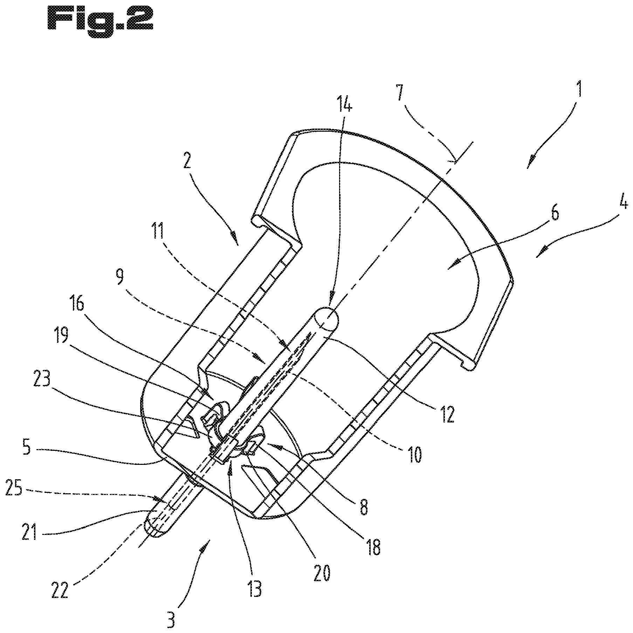

Unter gekoppelt wird hier verstanden, dass die Kanüle 10 am Hauptkörperteil 2 im Bereich seiner Frontwand 5 gehalten oder angeordnet ist. Dazu kann entweder die Kanüle 10 aus einem eigenen, unabhängigen Bauteil gebildet sein, welcher mit dem Hauptkörperteil 2 verbunden ist. Diese Ausbildung wird in den nachfolgenden Fig. 2 bis 4 gezeigt und beschrieben.

Andererseits wäre es aber auch möglich, wie dies hier in der Fig. 1 dargestellt ist, dass die Kanüle 10 als integraler Bestandteil des Hauptkörperteils 2 ausgebildet ist. Bevorzugt wird der Hauptkörperteil 2 mit seiner Frontwand 5 als einstückiger Bauteil in einem Spritzgießvorgang zumeist aus einem durchscheinenden bis durchsichtigen Kunststoffmaterial gebildet. So wäre es aber auch noch möglich, den Hauptkörperteil 2 mit seiner Frontwand 5 aus einem ersten Werkstoff und die Kanüle 10 aus einem dazu unterschiedlichen zweiten Werkstoff in Form eines Coinjektion-Spritzgusses herzustellen. Damit wird die Möglichkeit geschaffen, ohne weitere nachträgliche Fügevorgänge einen Grundkörper zu schaffen, an welchem in einem Herstellvorgang auch gleich die Kanüle 10 mit ausgebildet ist.

Um Stichverletzungen bzw. einen ungewollten Austritt von Körperflüssigkeiten, insbesondere Blut, aus dem proximalen Kanülenende 11 der Kanüle 10 zu vermeiden, kann in bekannter Weise dazu eine schlauchförmige, elastisch verformbare sowie durchstechbare Hülle 12 vorgesehen sein. Die Hülle 12, welche auch als Schlauchventil bezeichnet werden kann, weist ein offenes distales Hüllenende 13 sowie ein verschlossen ausgebildetes proximales Hüllenende 14 auf. Der Werkstoff der Hülle 12 ist bevorzugt aus einem durchstechbaren, selbstverschließenden Werkstoff gewählt, wie dies hinlänglich bekannt ist.

Die zuvor beschriebene Haltevorrichtung 8 dient dazu, die Hülle 12 im Bereich der Frontwand 5 positioniert zu halten. Dazu kann die Haltevorrichtung 8 einen in etwa zylindrisch oder kegelförmig ausgebildeten Zentrieransatz 15 umfassen, welcher direkt an der Frontwand 5 ausgebildet ist. Damit ist auch der Zentrieransatz 15 direkter, integraler Bestandteil der Frontwand 5 und somit des Hauptkörperteils 2.

Das offen ausgebildete distale Hüllenende 13 ist auf den Zentrieransatz 15 aufgesetzt und somit an diesem angeordnet bzw. gehalten. So ragt der Zentrieransatz 15 in das offene distale Hüllenende 13 der Hülle 12 hinein. Da der Werkstoff der Hülle 12 elastisch verformbar ist, wird zwischen dem Zentrieransatz 15 und dem offenen distalen Hüllenende 13 eine satte Anlage, insbesondere eine Presspassung gewählt. Dies erfolgt durch elastisches Aufweiten des distalen Hüllenendes 13, wie dies vereinfacht dargestellt ist. Weiters ist in dieser Position der Hülle 12 das proximale Kanülenende 11 der Kanüle 10 vom verschlossenen proximalen Hüllenende 14 der Hülle 12 abgedeckt.

Weiters umfasst die Haltevorrichtung 8 zumindest ein, bevorzugt jedoch mehrere, an der Frontwand 5 ausgebildete oder an der Frontwand 5 angeordnete Halteelemente 16 für die Hülle 12. Zumeist werden die Halteelemente 16 gleichmäßig über den Umfang verteilt angeordnet, wobei deren Anzahl zumeist zwischen zwei und acht Stück und auch mehr betragen kann. Das zumindest eine Halteelement 16 ist an der dem Aufnahmeraum 6 zugewendeten Seite der Frontwand 5 sowie in einer radialen Distanz unter Ausbildung eines Spaltes 17 vom Zentrieransatz 15 distanziert angeordnet. Der Spalt 17 oder die Spalte 17 dienen zur Aufnahme einer Hüllenwand der Hülle 12, um so im fertig gefügten Zustand eine relative Positionierung und/oder Halterung der Hülle 12 am Zentrieransatz 15 zu bewirken. Dabei liegt das zumindest eine Halteelement 16 außenseitig in einem Kontaktbereich an der Hülle 12 an. Dies ist vereinfacht durch eine Verformung im Anlagebereich der Halteelemente 16 an der Hülle 12 dargestellt. Dabei kann die radiale Distanz zur Ausbildung des Spaltes 17 zwischen dem zumindest einen Halteelement 16 und dem Zentrieransatz 15 bei an der Hülle 12 anliegendem Halteelement 16 einen Wert aufweisen, welcher aus einem Wertebereich stammt, dessen untere Grenze 5 % und dessen obere Grenze 95 % bezüglich der unverformten Wandstärke der Hülle 12 in diesem Bereich beträgt. Je geringer die Weite des Spalts 17 gewählt wird, desto höher ist die dabei erzielte Anpressung an den Zentrieransatz 15 und die damit verbundene Verformung der Hülle 12, insbesondere deren Hüllenwand.

Weiters ist hier noch gezeigt, dass das mindestens eine Halteelement 16 einen von der Frontwand 5 vorragenden Haltearm 18 und eine am Haltearm 18 in Richtung auf die Längsachse 7 und somit auf den Zentrieransatz 15 vorragende Haltenase 19 aufweist. Dabei liegt zumindest die Haltenase 19 des mindestens einen Halteelements 16 im Kontaktbereich an der Außenseite der Hüllenwand der Hülle 12 an. Je nach gewählter Spaltweite des Spalts 17 kann das Ausmaß der Klemmung der Hülle 12 zwischen dem Zentrieransatz 15 und dem zumindest einen Halteelement 16 festgelegt werden. Um einen Federarm, welcher durch den zumindest einen Haltearm 18 gebildet ist, ausbilden zu können, kann die Haltenase 19 in Axialrichtung von der Frontwand 5 distanziert angeordnet sein.

Zur einfacheren Herstellung und Entformung der Handhabungsvorrichtung 1, insbesondere dessen Haltevorrichtung 8, mittels eines einfachen Öffnungsprozesses des Spritzgusswerkzeuges, ist hier weiters noch vorgesehen, dass in der Frontwand 5 zwischen dem mindestens einen Halteelement 16, insbesondere dessen Haltearm 18, und dem Zentrieransatz 15 ein die Frontwand 5 durchsetzender Durchbruch 20 ausgebildet oder angeordnet ist. Je nach Anzahl der Halteelemente 16 ist dazu die entsprechend gleiche Anzahl an Durchbrüchen 20 zu wählen. Der Durchbruch 20 erstreckt sich ausgehend vom Randbereich des Haltearms 18 des Halteelements 16 zumindest soweit hin in Richtung auf die Längsachse 7, dass der vorderste, und somit der der Längsachse 7 zugewendete Randbereich der Haltenase 19 in Axialrichtung mit dem Durchbruch 20 fluchtet. Die Größe bzw. der Querschnitt des Durchbruchs 20 entspricht in Axialrichtung zumindest einer Projektionsfläche der Haltenase 19 sowie gegebenenfalls einem Anteil des Haltearms 18 bei einer möglichen Schrägstellung desselben. So kann in einer einfachen Öffnungsbewegung die Entformung der Handhabungsvorrichtung 1 aus dem nicht näher dargestellten Formgebungswerkzeug erfolgen.

Zur Vermeidung eines direkten Kontaktes des im Aufnahmeraum 6 aufzunehmenden Aufnahmebehälters, insbesondere dessen Verschlussvorrichtung, mit den Halteelementen 16 der Haltevorrichtung 8 zu vermeiden, kann oder können an der dem Aufnahmeraum 6 zugewendeten Innenseite der Frontwand 5 nicht näher bezeichnete Abstandshalteelemente angeordnet oder ausgebildet sein. Diese können durch Stege und/oder Rippen gebildet sein, welche es verhindern, dass der Aufnahmebehälter weiter als bis zu dessen Anlage und der damit verbundenen Abstützung an dem oder den Abstandshalteelementen eingeschoben werden kann. Damit kann einerseits eine Beschädigung der Halteelemente 16 verhindert und andererseits ein ausreichender Verformungsfreiraum für die Hülle 12 während des Durchstichs der Kanüle 10 durch die Verschlussvorrichtung des Aufnahmebehälters geschaffen werden.

In dem hier vorliegenden Ausführungsbeispiel bildet der Hauptkörperteil 2 mit der Frontwand 5 sowie der Haltevorrichtung 8 gemeinsam mit der Kanüle 10 einen zusammengehörigen Bauteil aus einem in einem Spritzgussprozess verarbeitbaren Werkstoff aus. So ist damit die Kanüle 10 direkt am Zentrieransatz 15 als integraler Bestandteil daran angeordnet und ausgebildet.

Des Weiteren ist hier noch gezeigt, dass an der Frontwand 5 an der vom Aufnahmeraum 6 abgewendeten Seite ein über die Frontwand 5 in Axialrichtung vorragendes Anschlussstück 21 angeordnet, insbesondere daran ausgebildet, sein kann.

Um eine Durchflussmöglichkeit zwischen dem distalen Endstück des Anschlussstücks 21 und dem proximalen Kanülenende 11 der Kanüle 10 zu ermöglichen, erstreckt sich durchgängig zwischen diesen Bauteilen ein Strömungskanal 22. Der Strömungskanal 22 ist damit innerhalb des Anschlussstücks 21 und des Zentrieransatzes 15 im Bereich der Frontwand 5 bis hin zur hohl ausgebildeten Kanüle 10 ausgebildet.

Das Anliegen des Halteelements 16 an der Außenseite der Hülle 12 in dem zuvor beschriebenen Kontaktbereich kann unterschiedlichst ausgeführt werden.

Eine erste Möglichkeit bestünde darin, das Halteelement 16, insbesondere dessen Haltearm 18, in einer Position relativ bezüglich des Zentrieransatzes 15 anzuordnen, sodass die Distanz und damit die Spaltweite des Spaltes 17 eine sichere Halterung und Klemmung der Hülle 12 durch das Halteelement 16 im Kontaktbereich am Zentrieransatz 15 bewirkt. Das elastisches Aufweiten und Verlagern des Halteelements 16, insbesondere dessen Haltearm 18, auf die von der Längsachse 7 abgewendete Seite ermöglicht das Aufsetzen und Positionieren der Hülle 12 mit ihrem distalen Hüllenende 13 am Zentrieransatz 15. Durch elastisches Rückfedern bzw. Rückstellen des zumindest einen Halteelements 16, insbesondere dessen Haltearm 18, erfolgt anschließend die klemmende Halterung der Hülle 12 am Zentrieransatz 15. Die verlagerte Stellung des Halteelements 16 ist im linken Teil der Haltevorrichtung 8 in strichlierten Linien angedeutet.

Eine zweite Möglichkeit bestünde darin, die Distanz und damit die Spaltweite des Spaltes 17 durch entsprechende Anordnung des zumindest einen Halteelements 16, insbesondere dessen Haltearm 18, so zu wählen, dass ein ungehindertes Aufsetzen, insbesondere Aufschieben, des offenen distalen Hüllenendes 13 auf den Zentrieransatz 15 ermöglicht wird. Anschließend daran wird durch einen entsprechenden Umformvorgang das zumindest eine Halteelement 16, insbesondere dessen Haltearm 18, soweit hin in Richtung auf die Längsachse 7 umgeformt bzw. verformt, dass dieses, insbesondere dessen Haltenase 19, außenseitig zur Anlage im Kontaktbereich an der Hülle 12 verbracht wird. Dies ist vereinfacht durch einen Pfeil im rechten Teil der Haltevorrichtung 8 angedeutet. So ist hier nur im rechten Teil die bereits umgeformte Position des Halteelements 16 gezeigt. Die unverformte Ausgangslage könnte dabei jene sein, wie diese im linken Teil der Haltevorrichtung 8 in strichlierten Linien angedeutet ist.

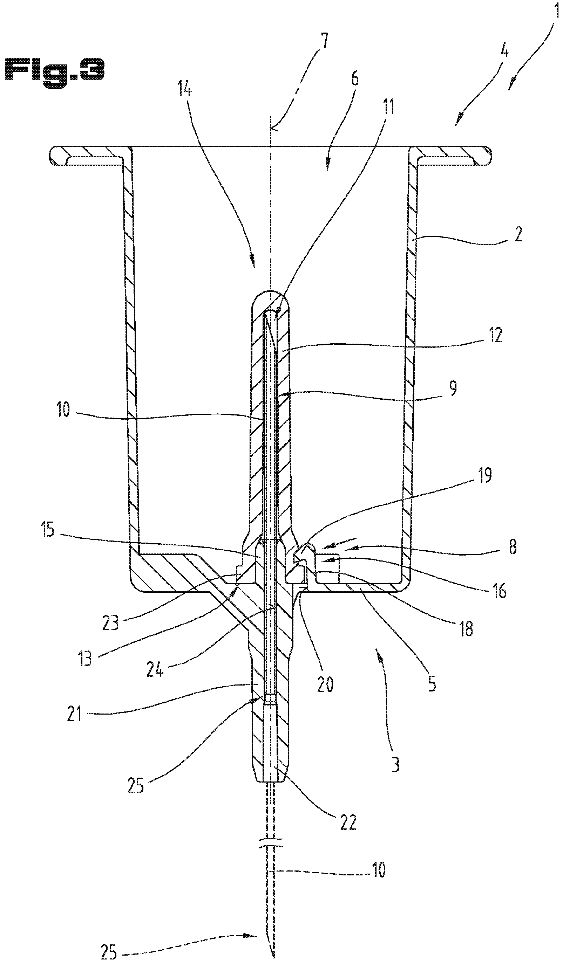

In den Fig. 2 und 3 ist eine weitere und gegebenenfalls für sich eigenständige Ausführungsform der Handhabungsvorrichtung 1 gezeigt, wobei wiederum für gleiche Teile gleiche Bezugszeichen bzw. Bauteilbezeichnungen wie in der vorangegangenen Fig. 1 verwendet werden. Um unnötige Wiederholungen zu vermeiden, wird auf die detaillierte Beschreibung in der vorangegangenen Fig. 1 hingewiesen bzw. Bezug genommen.

Auch bei dieser hier gezeigten Ausführungsform der Handhabungsvorrichtung 1 umfasst diese wiederum den Hauptkörperteil 2 mit dessen distalen und mit der Frontwand 5 verschlossenen Ende 3 sowie dem offen ausgebildeten, proximalen Ende 4. Die Längsachse 7 erstreckt sich zwischen diesen beiden Enden 3, 4. Der Zentrieransatz 15 ist ebenfalls direkter, integraler Bestandteil der Frontwand 5 und ragt von dieser in den Aufnahmeraum 6 vor. Weiters ist hier noch zusätzlich gezeigt, dass die Hülle 12 im Bereich ihres offenen distalen Hüllenendes 13 einen in radialer Richtung über die Hülle 12 vorragenden Kragen 23 aufweist. Der Kragen 23 dient im vorliegenden Ausführungsbeispiel als formschlüssiges Verbindungsmittel und/oder Haltemittel im Zusammenwirken mit der Haltevorrichtung 8, insbesondere dessen mindestens einen Halteelement 16. Um einen gegenseitigen formschlüssigen Eingriff zu erzielen, kann die Haltenase 19 des mindestens einen Halteelements 16 den Kragen 23 der Hülle 12 an seiner dem offen ausgebildeten, proximalen Ende 4 des Hauptkörperteils 2 zugewendeten Seite hintergreifen.

Wie bereits zuvor beschrieben, kann das Hintergreifen und das Anliegen an der Außenseite der Hülle 12 analog erfolgen, wie dies bereits zuvor in der Fig. 1 detailliert beschrieben worden ist. Weiters wäre es bei dieser Ausführung noch möglich, dass die Haltenase 19 lediglich den Kragen 23 hintergreift und keinen zusätzlichen Druck auf die Hüllenwand der Hülle 12 ausübt und die zuvor beschriebene Verformung der Hülle 12, insbesondere deren Hüllenwand nicht bewirkt. Es wäre aber auch wiederum möglich, das Halteelement 16, insbesondere dessen Haltearm 18, in seiner Lage für die Halterung der Hülle 12 umzuformen. Dies kann z.B. durch einen thermischen Umformvorgang erfolgen.

Im Gegensatz zu der zuvor beschriebenen Ausführungsform gemäß der Fig. 1 ist hier dargestellt, dass die Kanüle 10 durch einen eigenen Bauteil, insbesondere aus einem metallischen Werkstoff, gebildet ist. Dazu ist eine haltende und gasdichte Verbindung zwischen der Kanüle 10 und dem Hauptkörperteil 2, insbesondere dessen Frontwand 5 auszubilden. So ist hier vorgesehen, dass in der Frontwand 5 des Hauptkörperteils 2 eine entsprechende Aufnahmeöffnung 24 ausgebildet ist. In diese Aufnahmeöffnung 24 ist die Kanüle 10 direkt eingesetzt. Ist das zuvor beschriebene Anschlussstück 21 wiederum vorgesehen, kann sich die Aufnahmeöffnung 24 auch in dieses zumindest teilweise hinein erstrecken. Die Aufnahmeöffnung 24 kann auch im Zentrieransatz 15 angeordnet sein. Je nach gewünschter Klemmlänge der Kanüle 10 kann die Aufnahmeöffnung 24 im Bereich des Zentrieransatzes 15 eine bezüglich der Kanülenabmessung dazu größere Innenabmessung aufweisen. Damit kann das Einsetzen bzw. Einführen der Kanüle 10 in die Aufnahmeöffnung 24 erleichtert werden.

Um auf zusätzliche Verbindungsmittel, wie beispielsweise Klebstoffe oder dergleichen, verzichten zu können, kann die Kanüle 10 in die im Bereich der Frontwand 5 ausgebildete Aufnahmeöffnung 24 eingepresst sein. Um das Ansaugen von Fremdluft in diesem Bereich zu vermeiden, ist auf eine gasdichte Pressverbindung zwischen der Außenseite der Kanüle 10 und dem Hauptkörperteil 2, insbesondere dessen Frontwand 5, zu achten. Durch diesen einfachen Prozessschritt kann auf zusätzliche Prozessschritte, wie das Aufbringen und Aushärten von zusätzlichen Klebemitteln, verzichtet werden. Damit können eine höhere Prozesssicherheit sowie damit einhergehende Verschmutzungen der Anlage durch Klebstoffreste oder unbrauchbare Handhabungsvorrichtungen 1 durch undichte Klebeverbindungen vermieden werden.

Um eine zentrische Anordnung und Lage der Kanüle 10 bezüglich des Hauptkörperteils 2 zu erzielen, kann es vorteilhaft sein, wenn sowohl die Aufnahmeöffnung 24 für die Kanüle 10 als auch der Zentrieransatz 15 für die Hülle 12 jeweils in der gemeinsamen Längsachse 7 liegend angeordnet sind.

Die Kanüle 10 weist ihrerseits auch noch ein distales Kanülenende 25 auf. Das distale Kanülenende 25 ragt zumindest in den Zentrieransatz 15 sowie gegebenenfalls die Frontwand 5 hinein. Ist das Anschlussstück 21 ebenfalls noch vorgesehen, kann die Kanüle 10 mit ihrem distalen Kanülenende 25 zumindest teilweise in das Anschlussstück 21 hineinragen.

Unabhängig davon wäre es aber auch noch möglich, die Kanüle 10 mit einer dazu größeren Längserstreckung auszubilden, wie dies in der Fig. 3 in strichlierten Linien dargestellt ist. Damit kann die Kanüle 10 mit ihrem distalen Kanülenende 25 das Anschlussstück 21 in Axialrichtung überragen. Des Weiteren kann die Kanüle 10 an ihrem distalen Kanülenende 25 ein Einstichende zum Einstechen in ein nicht näher dargestelltes Körperteil aufweisen. Das Einstichende ist dabei meist durch eine Abschrägung an der Kanüle 10 ausgebildet.

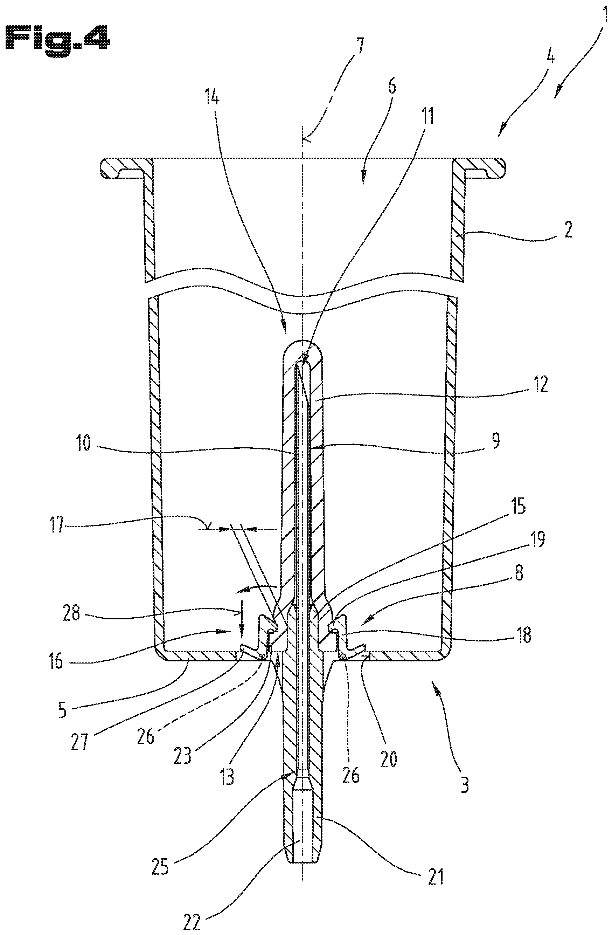

In der Fig. 4 ist eine weitere und gegebenenfalls für sich eigenständige Ausführungsform der Handhabungsvorrichtung 1 gezeigt, wobei wiederum für gleiche Teile gleiche Bezugszeichen bzw. Bauteilbezeichnungen wie in den vorangegangenen Fig. 1 bis 3 verwendet werden. Um unnötige Wiederholungen zu vermeiden, wird auf die detaillierte Beschreibung in den vorangegangenen Fig. 1 bis 3 hingewiesen bzw. Bezug genommen.

Bei diesem hier gezeigten Ausführungsbeispiel der Handhabungsvorrichtung 1 umfasst diese wiederum den Hauptkörperteil 2 mit dessen distalen und der Frontwand 5 verschlossenen Ende 3 sowie dem offen ausgebildeten, proximalen Ende 4. Im Bereich der Frontwand 5 ist wiederum der Zentrieransatz 15 angeordnet, der ebenfalls direkter, integraler Bestandteil der Frontwand 5 ist. Am Zentrieransatz 15 ist die Hülle 12 aufgesetzt, wie dies bereits zuvor beschrieben worden ist.

Auch bei diesem Ausführungsbeispiel weist die Hülle 12 im Bereich ihres offenen distalen Hüllenendes 13 den in radialer Richtung über die Hülle 12 vorragenden Kragen 23 auf. Der Kragen 23 dient auch bei hier als formschlüssiges Verbindungsmittel und/oder Haltemittel im Zusammenwirken mit der Haltevorrichtung 8, insbesondere dessen mindestens einen Halteelement 16.

Die Kanüle 10 ist auch bei diesem Ausführungsbeispiel durch einen eigenen Bauteil, insbesondere aus einem metallischen Werkstoff gebildet. Weiters ist die Kanüle 10 wiederum direkt mit dem Hauptkörperteil 2, insbesondere dessen Frontwand 5, sowie gegebenenfalls dem Anschlussstück 21 durch einen Presssitz verbunden. Um das Ansaugen von Fremdluft in diesem Bereich zu vermeiden, ist auf eine gasdichte Pressverbindung zwischen der Außenseite der Kanüle 10 und dem Hauptkörperteil 2, insbesondere dessen Frontwand 5, zu achten.

Die Haltevorrichtung 8 umfasst wiederum mindestens das eine Halteelement 16, welches im vorliegenden Ausführungsbeispiel an der Frontwand 5 relativ zu dieser verschwenkbar daran angeordnet ist. Das Halteelement 16 umfasst wiederum den von der Frontwand 5 aufragenden Haltearm 18 an welchem die Haltenase 19 in Richtung auf die Längsachse 7 bzw. den Zentrieransatz 15 darüber vorragend angeordnet sein kann. Der Haltearm 18 ist in seinem der Frontwand 5 zugewendeten Haltearmende bevorzugt beidseitig über ortsfeste Haltezapfen 26 mit der Frontwand 5 verbunden. Die Haltezapfen 26 überbrücken beidseits die Freistellung des Haltearms 18 im Bereich des Durchbruchs 20 und bilden jeweils für sich eine Art Torsionsstab aus. Damit wird es möglich, über die Haltezapfen 26 den Haltearm 18 in seiner relativen Lage bezüglich der Frontwand 5 elastisch und selbstrückstellend verlagern zu können. Der Haltearm 18 führt dabei um die Haltezapfen 26 eine Art Kipp- bzw. Schwenkbewegung durch. Aufgrund des Vorsehens des Durchbruchs 20 kann so wiederum eine einstückige Ausbildung des mindestens einen Halteelements 16 im Bereich der Frontwand 5 an dieser erfolgen. Da das zumindest eine Halteelement 16 auch hier direkt an der Frontwand 5 als integraler Bestandteil derselben und somit auch des Hauptkörperteils 2 ist, kann auf zusätzliche Fertigungs- und Fügeschritte verzichtet werden. Es wäre aber auch denkbar, dass das mindestens eine Halteelement 16 durch einen eigenen Bauteil gebildet ist, welcher an der Frontwand 5 befestigt ist.

Um die Verstellung bzw. die relative Verlagerung des Haltearms 18 beispielsweise für die Aufsetzbewegung der Hülle 12 auf den Zentrieransatz 15 zu erleichtern, kann am Haltearm 18 auf der vom Zentrieransatz 15 sowie von der Haltenase 19 abgewendeten Seite ein Stellarm 27 daran angeordnet oder ausgebildet sein. Der Stellarm 27 kann mit dem Haltearm 18 auf der dem Aufnahmeraum 6 zugewendeten Seite einen Winkel einschließen, welcher kleiner ausgebildet ist als 90°. Bevorzugt wird der eingeschlossene Winkel zwischen 80° und 50° gewählt. Bedingt durch diese Schrägstellung des Stellarms 27 relativ bezüglich dem Haltearm 18 und damit auch der Frontwand 5 ragt zumindest ein Teilabschnitt des Stellarms 27 über die Frontwand 5 in den Aufnahmeraum 6 vor. Zumeist wird in der unverformten Ausgangslage eine rechtwinkelige Ausrichtung des Haltearms 18 bezüglich der Frontwand 5 oder aber auch eine parallel verlaufende Ausrichtung bezüglich der Längsachse 7 gewählt.

Wird auf den Stellarm 27 eine Kraft - gemäß eingetragenem Pfeil 28 - auf den Stellarm 27 ausgeübt, wird der Stellarm 27 um die Haltezapfen 26 in Richtung auf die Frontwand 5 verschwenkt. Dadurch wird aber auch der Haltearm 18 gemeinsam mit der Haltenase 19 auf die von der Längsachse 7 abgewendete Seite verschwenkt, wodurch der Spalt 17 zwischen dem Haltearm 18, insbesondere dessen Haltenase 19, und dem Zentrieransatz 15 vergrößert wird. Dadurch kann das Aufsetzen der Hülle 12 auf den Zentrieransatz 15 auch bei daran angeordnetem Kragen 23 einfach erfolgen.

Bei Wegnahme der Kraft - gemäß Pfeil 28 - erfolgt ein selbsttätiges Rückstellen des Halteelements 16 in seine Ausgangslage. Hier kann wiederum das Hintergreifen des Kragens 23 durch die Haltenase 19 auf seiner dem offenen proximalen Ende 4 zugewendeten Seite erfolgen.

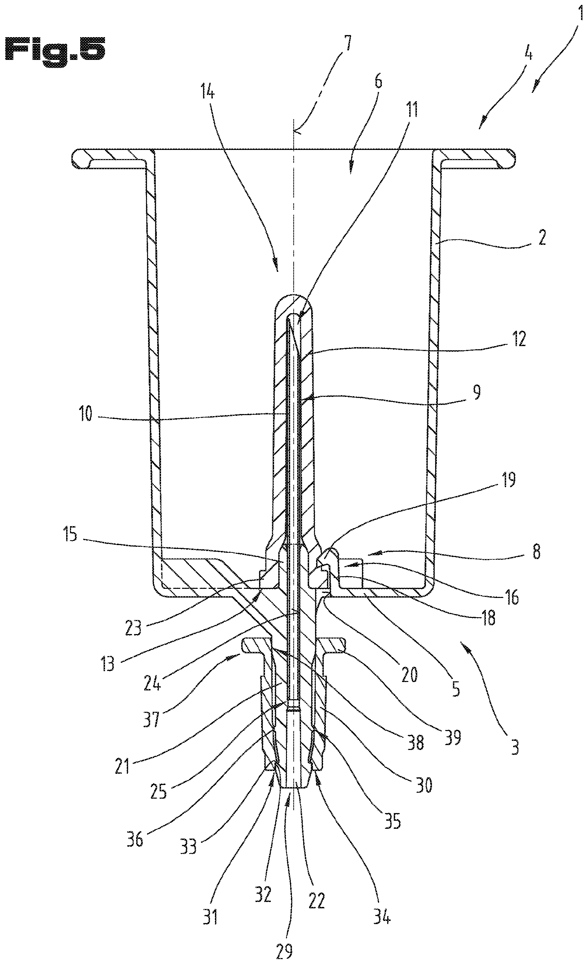

In der Fig. 5 ist eine weitere und gegebenenfalls für sich eigenständige Ausführungsform der Handhabungsvorrichtung 1 gezeigt, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Die Ausbildung der Haltevorrichtung 8 sowie die Ausbildung und Anordnung der Kanüle 10 sowie dessen Hülle 12 kann gemäß einer der zuvor beschriebenen Ausführungsformen erfolgen. Es können auch die unterschiedlichen Ausbildungen der zuvor beschriebenen Bauteile beliebig miteinander zu einer Einheit kombiniert werden. Damit kann diese hier beschriebene zusätzliche Ausführungsform mit jedem der zuvor beschrieben Ausführungsbeispiele kombiniert werden.

Das Anschlussstück 21 kann im Axialschnitt gesehen, die unterschiedlichsten Raumformen sowie Querschnittsformen aufweisen und es kann somit die Handhabungsvorrichtung 1 für die unterschiedlichen Konfigurationen adaptiert werden. Bei einer in Richtung des distalen Endes sich verjüngenden Ausbildung des Anschlussstücks 21 kann so einfach ein Durchstechen von Membranen, insbesondere Silikonmembranen, erfolgen, wie diese bei Blutbeuteln oder Plasmabeuteln Anwendung finden. Die Außenform des Anschlussstücks 21 kann auch durch einen in der Medizintechnik üblichen Luer-Konus gebildet sein, welcher auch als Luer-Ansatz bezeichnet werden kann, und einen Kegelwinkel von 6% aufweist. Damit können die unterschiedlichsten medizinischen Bauteile, wie z.B. Luernadeln, Schlauchanschlüsse oder dergleichen, einfach verbunden, insbesondere gekuppelt werden.

Das Anschlussstück 21 weist bei diesem Ausführungsbeispiel ausgehend von der Frontwand 5 hin zu seinem distalen Anschlussstückende 29 im Axialschnitt gesehen einen gestuft ausgebildeten Längsverlauf auf. Weiters dient das Anschlussstück 21 bei diesem Ausführungsbeispiel als Kupplungsstück mit einem Adapter 30. Der Adapter 30 dient als Zwischenstück, um so je nach Ausbildung des Adapters 30 über diesen eine Verbindungs- bzw. Kupplungsmöglichkeit mit dem Anschlussstück 21 und damit der Handhabungsvorrichtung 1 herstellen zu können. Der Adapter 30 kann vorzugsweise aus einem zum Werkstoff des Hauptkörperteils 2 bzw. dessen Frontwand 5 dazu unterschiedlichen Werkstoff gebildet sein. Bevorzugt wird ein durchsichtiger und/oder transparenter Werkstoff, insbesondere aus Kunststoff, zur Bildung des Hauptkörperteils 2 bzw. dessen Frontwand 5 und den an dieser angeordneten Bauteile der Haltevorrichtung 8 gewählt. Weiters kann entweder direkt mit dem Anschlussstück 21 oder mit dem Adapter 30 ein Schlauch verbunden werden. Der nicht näher dargestellte Schlauch kann auch aus einem Kunststoff, insbesondere PVC-Werkstoff, gebildet sein. Auch eine Verklebung mit dem Anschlussstück 21 oder mit dem Adapter 30 ist möglich.

Im Inneren des Anschlussstücks 21 kann wiederum teilweise hineinragend die Kanüle 10 über einen gasdichten Presssitz gehalten sein. Des Weiteren erstreckt sich der Strömungskanal 22 im Anschluss an die Kanüle 10 im Zentrum des Anschlussstücks 21 bis hin zum distalen offenen Anschlussstückende 29 des Anschlussstücks 21.

Zur gegenseitigen Halterung und axialen Lagefixierung des Adapters 30 am Anschlussstück 21 ist eine Rastvorrichtung 31 mit zusammenwirkenden Rastelementen 32, 33 vorgesehen. Die Rastvorrichtung 31 ist bei diesem Ausführungsbeispiel benachbart dem distalen Anschlussstückende 29 angeordnet. Das erste am Anschlussstück 21 ausgebildete bzw. angeordnete Rastelement 32 ist als umlaufender Vorsprung mit einer in Richtung auf die Frontwand 5 ausgebildeten Hinterschneidung realisiert. Das entsprechende Gegenstück, nämlich das zweite Rastelement 33, ist im offenen distalen Adapterende 34 angeordnet.

In einem Mittelabschnitt des Adapters 30 kann ein erster Dichtabschnitt 35 vorgesehen sein. Dieser erste Dichtabschnitt 35 ist dabei durch ein am Anschlussstück 21 umlaufend ausgebildetes und über die Außenfläche des Anschlussstücks 21 darüber vorragendes Dichtelement 36, zum Beispiel in Form eines Dichtrings, gebildet. Dieses in radialer Richtung vorragende Dichtelement 36 weist mit zunehmendem Abstand von der Längsachse 7 im Axialschnitt gesehen, eine abnehmende Querschnittsform auf.

Im Bereich eines proximalen Adapterendes 37 ist bei diesem Ausführungsbeispiel noch gezeigt, dass dort ein zweiter Dichtabschnitt 38 und/oder ein Zentrierabschnitt ausgebildet bzw. angeordnet sein kann. Aus Sicherheitsgründen können beide Dichtabschnitte 35, 38 vorgesehen sein, welche beide bevorzugt eine gasdichte Anlage aneinander ausbilden. So kann das Ansaugen von Fremdluft zwischen dem Adapter 30 und dem Anschlussstück 21 hin zum Strömungskanal 22 verhindert bzw. vermieden werden.

Weiters ist hier noch dargestellt, dass im Bereich des proximalen Adapterendes 37 ein Flansch 39 angeordnet bzw. ausgebildet sein kann. Der Flansch 39 kann beispielsweise dazu dienen, eine Anschlagbegrenzung für ein an der Außenseite des Adapters 30 angeordnetes und daran gehaltenes Schlauchstück oder dergleichen zu bilden. Weiters kann der Flansch 39 auch als Zuführhilfe und/oder zur radialen Orientierung, dem Transport sowie der Ausrichtung des Adapters 30 dienen.

Weiters ist hier noch dargestellt, dass es vorteilhaft sein kann, wenn zwischen den beiden Dichtabschnitten 35 und 38 eine Distanzierung der Innenfläche des Adapters 30 von der Außenfläche des Anschlussstücks 21 vorhanden ist. Damit kann die Aufsetzbewegung des Adapters 30 auf das Anschlussstück 21 erleichtert werden und lediglich in zumindest einem der beiden Dichtabschnitte 35 und/oder 38 eine sichere gasdichte Verbindung durch einen Presssitz geschaffen werden. Dabei sei erwähnt, dass es auch möglich ist, nur eine der beiden zuvor erwähnten Dichtabschnitte 35 oder 38 auszubilden bzw. vorzusehen.

Das Anschlussstück 21 sowie der daran in Axialrichtung arretiert bzw. gekuppelt gehaltene Adapter 30 kann auch bei allen anderen hier beschriebenen Ausführungsformen Anwendung finden. Gleiches gilt aber auch für die das Anschlussstück 21 in distaler Richtung überragende Kanüle 10.

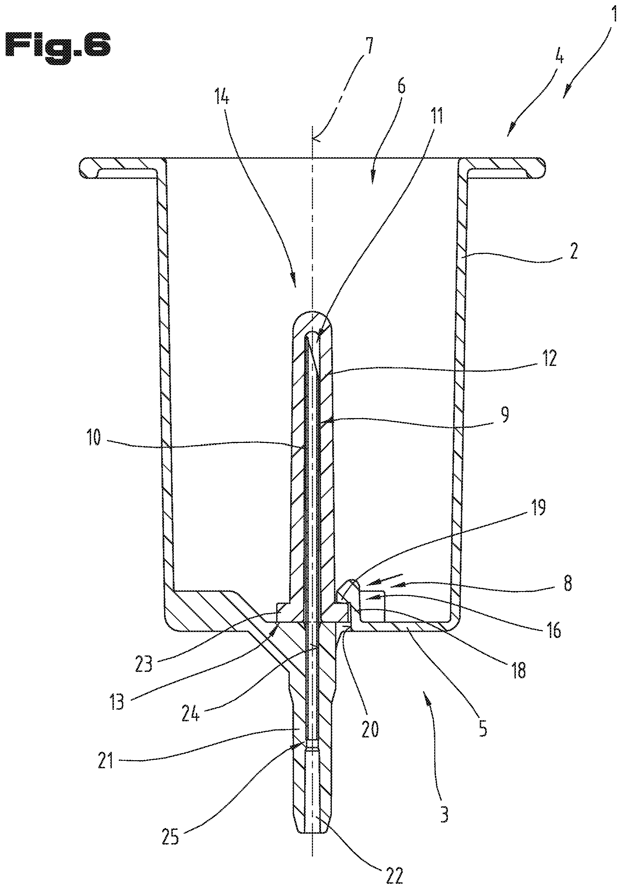

In der In der Fig. 6 ist eine weitere und gegebenenfalls für sich eigenständige Ausführungsform der Handhabungsvorrichtung 1 gezeigt, wobei wiederum für gleiche Teile gleiche Bezugszeichen bzw. Bauteilbezeichnungen wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen.

Da diese hier gezeigte Ausführungsform sehr ähnlich ist, wie jene gemäß der Fig. 2 und 3, wird hier nur auf die dazu unterschiedlichen Details näher eingegangen.

Auch bei dieser hier gezeigten Ausführungsform der Handhabungsvorrichtung 1 umfasst diese wiederum den Hauptkörperteil 2 mit dessen distalen und mit der Frontwand 5 verschlossenen Ende 3 sowie dem offen ausgebildeten, proximalen Ende 4. Die Längsachse 7 erstreckt sich zwischen diesen beiden Enden 3, 4.

Im Gegensatz zur Ausführung der Fig.2 und 3 ist hier die Frontwand 5 im Bereich der daran anzuordnenden Hülle 12 in etwa ebenflächig und somit ohne dem zuvor beschriebenen Zentrieransatz 15 ausgebildet. Die Hülle 12 kann den im Bereich ihres offenen distalen Hüllenendes 13 in radialer Richtung über die Hülle 12 vorragenden Kragen 23 aufweisen, ist jedoch dieser nicht zwingend notwendig. Der Kragen 23 dient im vorliegenden Ausführungsbeispiel dann als formschlüssiges Verbindungsmittel und/oder Haltemittel im Zusammenwirken mit der Haltevorrichtung 8, insbesondere dessen mindestens einen Halteelement 16. Um einen gegenseitigen formschlüssigen Eingriff zu erzielen, hintergreift die Haltenase 19 des mindestens einen Halteelements 16 den Kragen 23 der Hülle 12 an seiner dem offen ausgebildeten, proximalen Ende 4 des Hauptkörperteils 2 zugewendeten Seite.

Wie bereits zuvor beschrieben, kann das Hintergreifen und das Anliegen an der Außenseite der Hülle 12 analog erfolgen, wie dies bereits zuvor in der Fig. 2 und 3 detailliert beschrieben worden ist. Es wäre aber auch wiederum möglich, das Halteelement 16, insbesondere dessen Haltearm 18, in seiner Lage für die Halterung der Hülle 12 umzuformen. Dies kann z.B. durch einen thermischen Umformvorgang erfolgen. Unabhängig davon könnte aber auch das zumindest eine Halteelement 16 schwenkbar und/oder durch einen eigenen Bauteil gebildet sein, welcher ebenfalls direkt an oder innerhalb der Frontwand 5 angeordnet und an dieser ausgebildet ist, wie dies in der Fig. 4 beschrieben worden ist. Es wäre auch noch möglich, die Kanüle 10 als integralen Bestandteil der Frontwand 5 bzw. des Hauptkörperteils 2 auszubilden.

Die Kanüle 10 ist auch hier durch einen eigenen Bauteil, insbesondere aus einem metallischen Werkstoff, gebildet. Dazu ist eine haltende und gasdichte Verbindung zwischen der Kanüle 10 und dem Hauptkörperteil 2, insbesondere dessen Frontwand 5 auszubilden. Um auf zusätzliche Verbindungsmittel, wie beispielsweise Klebstoffe oder dergleichen, verzichten zu können, kann die Kanüle 10 in die im Bereich der Frontwand 5 ausgebildete Aufnahmeöffnung 24 eingepresst sein.

Es wäre aber auch möglich, dass die Kanüle 10 direkt an der Frontwand 5 als integraler Bestandteil des Hauptkörperteils 2 ausgebildet ist, wobei eine ähnliche Ausbildung in der Fig. 1, jedoch dort mit dem Zentrieransatz 15, beschrieben worden ist.

In Axialrichtung gesehen kann in einem Projektionsabschnitt des Halteelements 16 neben diesem in radialer Richtung auf die Längsachse 7 hin, auch hier der Durchbruch 20 in der Frontwand 5 angeordnet bzw. ausgebildet sein. Dieser dient auch hier zur Formgebung des mindestens einen Halteelements 16. Der Durchbruch 20 erstreckt sich ausgehend vom Randbereich des Haltearms 18 des Halteelements 16 zumindest soweit hin in Richtung auf die Längsachse 7, dass der vorderste, und somit der der Längsachse 7 zugewendete Randbereich der Haltenase 19 in Axialrichtung mit dem Durchbruch 20 fluchtet.

Die Hülle 12 liegt mit ihrem offenen distalen Hüllenende 13 an der Frontwand 5 abstützend daran in Axialrichtung an und wird durch das zumindest eine Halteelement 16 sowohl in Axialrichtung als auch in einem gewissen Ausmaß in Radialer Richtung an der Frontwand 5 gehalten. Dazu ist das Zusammenwirken mit dem Kragen 23 vorgesehen.

Die Herstellung bzw. Bereitstellung und das Fügen der einzelnen Bauteile zur Handhabungsvorrichtung 1 kann je nach Ausbildung des Grundkörpers bzw. Basiskörpers derselben etwas voneinander abweichende bzw. unterschiedliche Verfahrens schritte umfassen:
- Ausbilden einer schlauchförmigen, elastisch verformbaren sowie durchstechbaren Hülle 12 mit einem offenen distalen Hüllenende 13 sowie einem verschlossenen proximalen Hüllenende 14;
- Ausbilden eines hohlen Hauptkörperteils 2 mit einem mit einer Frontwand 5 zumindest teilweise verschlossenen distalen Ende 3 und einem offenen proximalen Ende 4, bei dem vom Hauptkörperteil 2 sowie von der Frontwand 5 ein Aufnahmeraum 6 definiert wird, und das proximale Ende 4 zur Aufnahme zumindest eines Teilabschnitts eines Aufnahmebehälters im Aufnahmeraum 6 dient, wobei sich zwischen dem distalen Ende 3 und dem proximalen Ende 4 eine Längsachse 7 erstreckt;
- Ausbilden einer Haltevorrichtung 8 für die Hülle 12 direkt im Bereich der Frontwand 5 an ihrer dem Aufnahmeraum 6 zugewendeten Seite, wobei die Haltevorrichtung 8 durch zumindest ein an der Frontwand 5 ausgebildetes oder an der Frontwand 5 angeordnetes Halteelement 16 gebildet wird;
- Anordnen und Verbinden einer als Kanüle 10 ausgebildeten Nadelanordnung 9 mit der Frontwand 5 des Hauptkörperteils 2 oder Ausbilden einer als Kanüle 10 ausgebildeten Nadelanordnung 9 an der Frontwand 5 des Hauptkörperteils 2, sodass die Kanüle 10 ausgehend von der Frontwand 5 mit ihrem proximalen Kanülenende 11 in den Aufnahmeraum 6 hineinragt;
- Verbringen der Hülle 12 mit ihrem offenen distalen Hüllenende 13 in den Bereich des zumindest einen an der Frontwand 5 ausgebildeten oder an der Frontwand 5 angeordneten Halteelements 16 und dabei Abdecken der in den Aufnahmeraum 6 hineinragenden Kanüle 10;
- Herstellen eines Kontaktbereichs zwischen dem Halteelement 16 und der Hülle 12, in welchem das Halteelement 16 außenseitig an der Hülle 12 zur Anlage gebracht wird.

Die Hülle 12 wird dabei bevorzugt im Bereich ihres offenen distalen Hüllenendes 13 mit dem in radialer Richtung über die Hülle 12 vorragenden Kragen 23 ausgebildet.

Des Weiteren kann an der Frontwand 5 als weiterer Bauteil der Haltevorrichtung 8 ein in etwa zylindrisch oder kegelstumpfförmig ausgebildeten Zentrieransatz 15 als integraler Bestandteil der Frontwand 5)ausgebildet werden. Der Zentrieransatz 15 wird in das offene distale Hüllenende 13 der Hülle 12 hineinragend ausgebildet.

Weiters ist es noch möglich, dass der zwischen dem zumindest einen Halteelement 16 und dem Zentrieransatz 15 ausgebildete Spalt 17 vor dem Aufsetzen der Hülle 12 auf den Zentrieransatz 15 mit einer bezüglich der Wandstärke der Hülle 12 dazu größeren Distanz ausgebildet wird. Nach dem Verbringen der Hülle 12 in den Spalt 17 wird das mindestens eine Halteelement 16 hin in Richtung auf die Längsachse 7 verlagert und damit außenseitig an der Hülle 12 im Kontaktbereich zur Anlage gebracht.

Die Ausführungsbeispiele zeigen mögliche Ausführungsvarianten der Handhabungsvorrichtung 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Vor allem können die einzelnen in den Fig. 1; 2, 3; 4; 5; 6 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

Der Ordnung halber sei abschließend darauf hingewiesen, dass zum besseren Verständnis des Aufbaus der Handhabungsvorrichtung 1 diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Handhabungsvorrichtung | 31 | Rastvorrichtung |
| 2 | Hauptkörperteil | 32 | Rastelement |
| 3 | distales Ende | 33 | Rastelement |
| 4 | proximales Ende | 34 | distales Adapterende |
| 5 | Frontwand | 35 | erster Dichtabschnitt |
| 6 | Aufnahmeraum | 36 | Dichtelement |
| 7 | Längsachse | 37 | proximales Adapterende |
| 8 | Haltevorrichtung | 38 | zweiter Dichtabschnitt |
| 9 | Nadelanordnung | 39 | Flansch |
| 10 | Kanüle | | |
| 11 | proximales Kanülenende | | |
| 12 | Hülle | | |
| 13 | distales Hüllenende | | |
| 14 | proximales Hüllenende | | |
| 15 | Zentrieransatz | | |
| 16 | Halteelement | | |
| 17 | Spalt | | |
| 18 | Haltearm | | |
| 19 | Haltenase | | |
| 20 | Durchbruch | | |
| 21 | Anschlussstück | | |
| 22 | Strömungskanal | | |
| 23 | Kragen | | |
| 24 | Aufnahmeöffnung | | |
| 25 | distales Kanülenende | | |
| 26 | Haltezapfen | | |
| 27 | Stellarm | | |
| 28 | Pfeil | | |
| 29 | distales Anschlussstückende | | |
| 30 | Adapter | | |

## Patentansprüche

1. Handhabungsvorrichtung (1), insbesondere für die Medizintechnik, mit
- einem hohl ausgebildeten Hauptkörperteil (2), welcher ein mit einer Frontwand (5) zumindest teilweise verschlossenes distales Ende (3) und ein offen ausgebildetes proximales Ende (4) aufweist, wobei der Hauptkörperteil (2) sowie die Frontwand (5) einen Aufnahmeraum (6) definieren und das proximale Ende (4) zur Aufnahme zumindest eines Teilabschnitts eines Aufnahmebehälters im Aufnahmeraum (6) ausgebildet ist, wobei sich zwischen dem distalen Ende (3) und dem proximalen Ende (4) eine Längsachse (7) erstreckt,
- einer als Kanüle (10) ausgebildeten Nadelanordnung (9), welche Kanüle (10) ausgehend von der Frontwand (5) mit ihrem proximalen Kanülenende (11) in den Aufnahmeraum (6) hineinragt, wobei die Kanüle (10) mit dem Hauptkörperteil (2) gekoppelt ist,
- einer schlauchförmigen, elastisch verformbaren sowie durchstechbaren Hülle (12), die ein offenes distales Hüllenende (13) sowie ein verschlossenes proximales Hüllenende (14) aufweist, und mit ihrem verschlossenen proximalen Hüllenende (14) das proximale Kanülenende (11) abdeckt,
- und mit einer Haltevorrichtung (8), mit welcher die Hülle (12) in Axialrichtung gehalten ist, wobei die Haltevorrichtung (8) für die Hülle (12) direkt an der Frontwand (5) ausgebildet oder angeordnet ist und dass die Haltevorrichtung (8) zumindest ein an der Frontwand (5) ausgebildetes oder an der Frontwand (5) angeordnetes Halteelement (16) für die Hülle (12) umfasst, wobei das zumindest eine Halteelement (16) an der dem Aufnahmeraum (6) zugewendeten Seite der Frontwand (5) angeordnet ist und einen von der Frontwand (5) vorragenden Haltearm (18) aufweist, und dass das zumindest eine Halteelement (16) au-ßenseitig in einem Kontaktbereich an der Hülle (12) anliegt,
**dadurch gekennzeichnet, dass**
- die Kanüle (10) direkt mit der Frontwand (5) des Hauptkörperteils (2) verbunden ist, oder dass die Kanüle (10) an der Frontwand (5) als integraler Bestandteil des Hauptkörperteils (2) ausgebildet ist,
- das mindestens eine Halteelement (16) eine am Haltearm (18) angeordnete sowie in Richtung auf die Längsachse (7) vorragende Haltenase (19) aufweist,
- in der Frontwand (5) in einem in Axialrichtung gesehen liegenden Projektionsabschnitt des zumindest einen Halteelements (16) neben diesem in radialer Richtung auf die Längsachse (7) hin, ein die Frontwand (5) durchsetzender Durchbruch (20) ausgebildet ist, und ein Querschnitt des Durchbruchs (20) in Axialrichtung zumindest einer Projektionsfläche der Haltenase (19) entspricht.

2. Handhabungsvorrichtung (1) nach Anspruch 1, **dadurch gekennzeichnet, dass** die Haltevorrichtung (8) einen in etwa zylindrisch oder kegelstumpfförmig ausgebildeten Zentrieransatz (15) aufweist, welcher integraler Bestandteil der Frontwand (5) ist, und der Zentrieransatz (15) in das offene distale Hüllenende (13) der Hülle (12) hineinragt.

3. Handhabungsvorrichtung (1) nach Anspruch 2, **dadurch gekennzeichnet, dass** das zumindest eine Halteelement (16) in einer radialen Distanz unter Ausbildung eines Spaltes (17) vom Zentrieransatz (15) angeordnet ist.

4. Handhabungsvorrichtung (1) nach Anspruch 3, **dadurch gekennzeichnet, dass** die radiale Distanz zwischen dem zumindest einen Halteelement (16) und dem Zentrieransatz (15) bei anliegendem Halteelement (16) an der Hülle (12) einen Wert aufweist, welcher aus einem Wertebereich stammt, dessen untere Grenze 5% und dessen obere Grenze 95% bezüglich der unverformten Wandstärke der Hülle (12) beträgt.

5. Handhabungsvorrichtung (1) nach Anspruch 1, **dadurch gekennzeichnet, dass** zumindest die Haltenase (19) des mindestens einen Halteelements (16) im Kontaktbereich an einer Hüllenwand der Hülle (12) anliegt.

6. Handhabungsvorrichtung (1) nach Anspruch 1 oder 5, **dadurch gekennzeichnet, dass** die Haltenase (19) in Axialrichtung von der Frontwand (5) distanziert angeordnet ist.

7. Handhabungsvorrichtung (1) nach Anspruch 2, **dadurch gekennzeichnet, dass** der die Frontwand (5) durchsetzende Durchbruch (20) zwischen dem Haltearm (18) des zumindest einen Halteelements (16) und dem Zentrieransatz (15) ausgebildet ist.

8. Handhabungsvorrichtung (1) nach einem der vorhergehenden Ansprüche, **dadurch gekennzeichnet, dass** die Hülle (12) im Bereich ihres offenen distalen Hüllenendes (13) einen in radialer Richtung über die Hülle (12) vorragenden Kragen (23) aufweist.

9. Handhabungsvorrichtung (1) nach einem der Ansprüche 5 bis 8 **dadurch gekennzeichnet, dass** die Haltenase (19) des mindestens einen Halteelements (16) den Kragen (23) der Hülle (12) an seiner dem offen ausgebildeten, proximalen Ende (4) des Hauptkörperteils (2) zugewendeten Seite hintergreift.

10. Handhabungsvorrichtung (1) nach einem der vorhergehenden Ansprüche, **dadurch gekennzeichnet, dass** das mindestens eine Halteelement (16) verstellbar, insbesondere verschwenkbar, an der Frontwand (5) gehalten ist.

11. Handhabungsvorrichtung (1) nach Anspruch 1, **dadurch gekennzeichnet, dass** die direkt mit der Frontwand (5) des Hauptkörperteils (2) verbundene Kanüle (10) in eine im Bereich der Frontwand (5) ausgebildete Aufnahmeöffnung (24) eingepresst ist.

12. Handhabungsvorrichtung (1) nach einem der vorhergehenden Ansprüche, **dadurch gekennzeichnet, dass** sowohl die Aufnahmeöffnung (24) für die Kanüle (10) als auch der Zentrieransatz (15) für die Hülle (12) jeweils in der Längsachse (7) liegend angeordnet sind.

13. Handhabungsvorrichtung (1) nach Anspruch 2, **dadurch gekennzeichnet, dass** die Kanüle (10) am Zentrieransatz (15) als integraler Bestandteil des Hauptkörperteils (2) ausgebildet ist.

14. Handhabungsvorrichtung (1) nach einem der vorhergehenden Ansprüche, **dadurch gekennzeichnet, dass** an der Frontwand (5) an der vom Aufnahmeraum (6) abgewendeten Seite ein über die Frontwand (5) in Axialrichtung vorragendes Anschlussstück (21) ausgebildet ist.

15. Handhabungsvorrichtung (1) nach Anspruch 14, **dadurch gekennzeichnet, dass** die Kanüle (10) mit ihrem distalen Kanülenende (25) zumindest teilweise in das Anschlussstück (21) hineinragt.

16. Handhabungsvorrichtung (1) nach Anspruch 14 oder 15, **dadurch gekennzeichnet, dass** die Kanüle (10) mit ihrem distalen Kanülenende (25) das Anschlussstück (21) in Axialrichtung überragt und an ihrem distalen Kanülenende (25) ein Einstichende zum Einstechen in einen Körperteil aufweist.

17. Handhabungsvorrichtung (1) nach einem der Ansprüche 14 bis 16, **dadurch gekennzeichnet, dass** am Anschlussstück (21) ein Adapter (30) positioniert gehalten ist und zwischen dem Anschlussstück (21) und dem Adapter (30) zumindest ein Dichtabschnitt (35, 38) ausgebildet ist.

18. Verfahren zur Herstellung einer Handhabungsvorrichtung (1), insbesondere für die Medizintechnik, das folgende Schritte umfasst:
- Ausbilden einer schlauchförmigen, elastisch verformbaren sowie durchstechbaren Hülle (12) mit einem offenen distalen Hüllenende (13) sowie einem verschlossenen proximalen Hüllenende (14);
- Ausbilden eines hohlen Hauptkörperteils (2) mit einem mit einer Frontwand (5) zumindest teilweise verschlossenen distalen Ende (3) und einem offenen proximalen Ende (4), bei dem vom Hauptkörperteil (2) sowie von der Frontwand (5) ein Aufnahmeraum (6) definiert wird, und das proximale Ende (4) zur Aufnahme zumindest eines Teilabschnitts eines Aufnahmebehälters im Aufnahmeraum (6) dient, wobei sich zwischen dem distalen Ende (3) und dem proximalen Ende (4) eine Längsachse (7) erstreckt;
- Ausbilden einer Haltevorrichtung (8) für die Hülle (12) direkt im Bereich der Frontwand (5) an ihrer dem Aufnahmeraum (6) zugewendeten Seite, wobei die Haltevorrichtung (8) durch zumindest ein an der Frontwand (5) ausgebildetes oder an der Frontwand (5) angeordnetes Halteelement (16) gebildet wird, wobei das zumindest eine Halteelement (16) durch einen von der Frontwand (5) vorragenden Haltearm (18) gebildet wird;
- Ausbilden einer Haltenase (19) am Haltearm (18) des mindestens einen Halteelements (16), wobei die Haltenase (19) in Richtung auf die Längsachse (7) vorragend ausgebildet wird;
- Ausbilden eines die Frontwand (5) durchsetzenden Durchbruchs (20), wobei der Durchbruch (20) in einem in Axialrichtung gesehen liegenden Projektionsabschnitt des zumindest einen Halteelements (16) neben diesem in radialer Richtung auf die Längsachse (7) hin ausgebildet wird und ein Querschnitt des Durchbruchs (20) in Axialrichtung zumindest mit einer der Haltenase (19) entsprechenden Projektionsfläche ausgebildet wird;
- Anordnen und Verbinden einer als Kanüle (10) ausgebildeten Nadelanordnung (9) direkt mit der Frontwand (5) des Hauptkörperteils (2) oder Ausbilden einer als Kanüle (10) ausgebildeten Nadelanordnung (9) an der Frontwand (5) als integraler Bestandteil des Hauptkörperteils (2), sodass die Kanüle (10) ausgehend von der Frontwand (5) mit ihrem proximalen Kanülenende (11) in den Aufnahmeraum (6) hineinragt;
- Verbringen der Hülle (12) mit ihrem offenen distalen Hüllenende (13) in den Bereich des zumindest einen an der Frontwand (5) ausgebildeten oder an der Frontwand (5) angeordneten Halteelements (16) und dabei Abdecken der in den Aufnahmeraum (6) hineinragenden Kanüle (10);
- Herstellen eines Kontaktbereichs zwischen dem Halteelement (16) und der Hülle (12), in welchem das Halteelement (16) außenseitig an der Hülle (12) zur Anlage gebracht wird.

19. Verfahren nach Anspruch 18, **dadurch gekennzeichnet, dass** an der Frontwand (5) als weiterer Bauteil der Haltevorrichtung (8) ein in etwa zylindrisch oder kegelstumpfförmig ausgebildeten Zentrieransatz (15) als integraler Bestandteil der Frontwand (5) ausgebildet wird, welcher Zentrieransatz (15) in das offene distale Hüllenende (13) der Hülle (12) hineinragend ausgebildet wird.

20. Verfahren nach Anspruch 19, **dadurch gekennzeichnet, dass** das zumindest eine Halteelement (16) in einer radialen Distanz unter Ausbildung eines Spaltes (17) vom Zentrieransatz (15) angeordnet wird.

21. Verfahren nach Anspruch 19, **dadurch gekennzeichnet, dass** der die Frontwand (5) durchsetzende Durchbruch (20) zwischen dem Haltearm (18) des zumindest einen Halteelements (16) und dem Zentrieransatz (15) ausgebildet wird.

22. Verfahren nach einem der Ansprüche 18 bis 21, **dadurch gekennzeichnet, dass** die Hülle (12) im Bereich ihres offenen distalen Hüllenendes (13) mit einen in radialer Richtung über die Hülle (12) vorragenden Kragen (23) ausgebildet wird.

23. Verfahren nach Anspruch 20 oder 22, **dadurch gekennzeichnet, dass** der zwischen dem zumindest einen Halteelement (16) und dem Zentrieransatz (15) ausgebildete Spalt (17) vor dem Aufsetzen der Hülle (12) auf den Zentrieransatz (15) mit einer bezüglich der Wandstärke der Hülle (12) dazu größeren Distanz ausgebildet wird und nach dem Verbringen der Hülle (12) in den Spalt (17) das mindestens eine Halteelement (16) hin in Richtung auf die Längsachse (7) verlagert und damit außenseitig an der Hülle (12) im Kontaktbereich zur Anlage gebracht wird.

## Claims

1. A handling device (1), in particular for medical technology, with
- a hollow main body part (2), which has a distal end (3) at least partially closed by a front wall (5) and an open proximal end (4), wherein the main body part (2) and the front wall (5) define a receiving space (6), and the proximal end (4) acts to receive at least one partial section of a receiving container in the receiving space (6), wherein a longitudinal axis (7) extends between the distal end (3) and the proximal end (4),
- a needle device (9) formed as a cannula (10), which cannula (10) protrudes into the receiving space (6) with its proximal cannula end (11) starting from the front wall (5), wherein the cannula (10) is coupled to the main body part (2),
- a hose-shaped, elastically deformable and perforable sleeve (12) that has an open distal sleeve end (13) and a closed proximal sleeve end (14) and covers the proximal cannula end (11) with its closed proximal sleeve end (14),
- and with a holding device (8) by which the sleeve (12) is held in the axial direction, wherein the holding device (8) for the sleeve (12) is formed or arranged directly on the front wall (5), and that the holding device (8) comprises at least one holding element (16) for the sleeve (12) formed on the front wall (5) or arranged on the front wall (5), wherein the at least one holding element (16) is arranged on the side of the front wall (5) facing the receiving space (6) and has a holding arm (18) protruding from the front wall (5), and that the at least one holding element (16) bears externally on the sleeve (12) in a contact area,
**characterized in that**
- the cannula (10) is directly connected to the front wall (5) of the main body part (2) or the cannula (10) is formed on the front wall (5) as an integrated part of the main body part (2),
- the at least one holding element (16) has a retaining collar (19) arranged on the holding arm (18) and protruding in the direction towards the longitudinal axis (7),
- in the front wall (5), in a projection section of the at least one holding element (16) seen in the axial direction, a perforation (20) through the front wall (5) is formed next to this element in the radial direction towards the longitudinal axis (7), and a cross-section of the perforation (20) in the axial direction corresponds to at least one projection area of the retaining collar (19).

2. The handling device (1) according to claim 1, **characterized in that** the holding device (8) has a roughly cylindrical or frustum-shaped centering projection (15) which is an integrated part of the front wall (5) and the centering projection (15) protrudes into the open distal sleeve end (13) of the sleeve (12).

3. The handling device (1) according to claim 2, **characterized in that** the at least one holding element (16) is arranged at a radial distance while forming a gap (17) from the centering projection (15).

4. The handling device (1) according to claim 3, **characterized in that** the radial distance between the at least one holding element (16) and the centering projection (15) when the holding element (16) is bearing on the sleeve (12) has a value within a range the lower limit of which is 5% and the upper limit of which is 95% of the non-deformed wall thickness of the sleeve (12).

5. The handling device (1) according to claim 1, **characterized in that** at least the retaining collar (19) of the at least one holding element (16) bears on the contact area of a sleeve wall of the sleeve (12).

6. The handling device (1) according to claim 1 or 5, **characterized in that** the retaining collar (19) is arranged at a distance from the front wall (5) in the axial direction.

7. The handling device (1) according to claim 2, **characterized in that** the perforation (20) through the front wall (5) is formed between the holding arm (18) of the at least one holding element (16) and the centering projection (15).

8. The handling device (1) according to one of the preceding claims, **characterized in that** in the region of its open distal sleeve end (13), the sleeve (12) has a lip (23) protruding beyond the sleeve (12) in the radial direction.

9. The handling device (1) according to one of claims 5 to 8, **characterized in that** the retaining collar (19) of the at least one holding element (16) engages behind the lip (23) of the sleeve (12) at its side that faces the open proximal end (4) of the main body part (2).

10. The handling device (1) according to one of the preceding claims, **characterized in that** the at least one holding element (16) is held on the front wall (5) such that it can be adjusted, in particular pivoted.

11. The handling device (1) according to claim 1, **characterized in that** the cannula (10) that is directly connected to the front wall (5) of the main body part (2) is pressed into a receiving opening (24) formed in the region of the front wall (5).

12. The handling device (1) according to one of the preceding claims, **characterized in that** both the receiving opening (24) for the cannula (10) and the centering projection (15) for the sleeve (12) are arranged lying on the longitudinal axis (7).

13. The handling device (1) according to claim 2, **characterized in that** the cannula (10) is formed on the centering projection (15) as an integrated part of the main body part (2).

14. The handling device (1) according to one of the preceding claims, **characterized in that** on the front wall (5) on the side facing away from the receiving space (6) a connecting piece (21) is formed that protrudes beyond the front wall (5) in the axial direction.

15. The handling device (1) according to claim 14, **characterized in that** the cannula (10) at least partially extends into the connecting piece (21) with its distal cannula end (25).

16. The handling device (1) according to claim 14 or 15, **characterized in that** the cannula (10) protrudes beyond the connecting piece (21) in the axial direction with its distal cannula end (25) and has a penetrating end at its distal cannula end (25) for penetrating into a body part.

17. The handling device (1) according to one of claims 14 to 16, **characterized in that** an adapter (30) is positioned on the connecting piece (21) and at least one sealing section (35, 38) is formed between the connecting piece (21) and the adapter (30).

18. A method for producing a handling device (1), in particular for medical technology, that comprises the following steps:
- formation of a hose-shaped, elastically deformable and perforable sleeve (12) with an open distal sleeve end (13) and a closed proximal sleeve end (14);
- formation of a hollow main body part (2) with a distal end (3) at least partially closed by a front wall (5) and an open proximal end (4), wherein the main body part (2) and the front wall (5) define a receiving space (6), and the proximal end (4) acts to receive at least one partial section of a receiving container in the receiving space (6), wherein a longitudinal axis (7) extends between the distal end (3) and the proximal end (4);
- formation of a holding device (8) for the sleeve (12) directly in the region of the front wall (5) on the side facing the receiving space (6), wherein the holding device (8) is formed by at least one holding element (16) formed on the front wall (5) or arranged on the front wall (5), wherein the at least one holding element (16) is formed by a holding arm (18) protruding from the front wall (5);
- formation of a retaining collar (19) on the holding arm (18) of the at least one holding element (16), wherein the retaining collar (19) is formed protruding in the direction towards the longitudinal axis (7);
- formation of a perforation (20) through the front wall (5), wherein the perforation (20), as seen in a projection section of the at least one holding element (16) in the axial direction, is formed next to this element in the radial direction towards the longitudinal axis (7) and a cross-section of the perforation (20) in the axial direction is formed with a projection area corresponding to at least one projection area of the retaining collar (19);
- arrangement and connection of a needle device (9) formed as a cannula (10) directly with the front wall (5) of the main body part (2) or formation of a needle device (9) formed as a cannula (10) on the front wall (5) of the main body part (2) as an integrated part such that the cannula (10) protrudes into the receiving space (6) with its proximal cannula end (11) starting from the front wall (5);
- placement of the sleeve (12) with its open distal sleeve end (13) in the region of the at least one holding element (16) formed on the front wall (5) or arranged on the front wall (5) and thereby coverage of the cannula (10) extending into the receiving space (6);
- production of a contact area between the holding element (16) and the sleeve (12) in which the holding element (16) bears externally on the sleeve (12).

19. The method according to claim 18, **characterized in that**, as an additional part of the holding device (8) on the front wall (5), a roughly cylindrical or frustum-shaped centering projection (15) is formed as an integrated part of the front wall (5), which centering projection (15) is formed to extend into the open distal sleeve end (13) of the sleeve (12).

20. The method according to claim 19, **characterized in that** the at least one holding element (16) is arranged at a radial distance while forming a gap (17) from the centering projection (15).

21. The method according to claim 19, **characterized in that** the perforation (20) through the front wall (5) is formed between the holding arm (18) of the at least one holding element (16) and the centering projection (15).

22. The method according to one of claims 18 to 21, **characterized in that** in the region of its open distal sleeve end (13), the sleeve (12) is formed with a lip (23) protruding beyond the sleeve (12) in the radial direction.

23. The method according to claim 20 or 22, **characterized in that** the gap (17) which is formed between the at least one holding element (16) and the centering projection (15) is of a distance larger than the wall thickness of the sleeve (12) before the sleeve (12) is placed over the centering projection (15) and, after the sleeve (12) is moved into the gap (17), the at least one holding element (16) is shifted in the direction towards the longitudinal axis (7) and therefore brought to bear externally on the sleeve (12) in the contact area.

## Revendications

1. Dispositif de manipulation (1), en particulier pour la technique médicale, avec
- une partie de corps principale (2) constituée de façon creuse qui comporte une extrémité distale (3) au moins partiellement fermée avec une paroi frontale (5) et une extrémité proximale (4) constituée de façon ouverte, dans lequel la partie de corps principale (2) ainsi que la paroi frontale (5) définissent un espace de réception (6), et l'extrémité proximale (4) est constituée pour la réception d'au moins un tronçon partiel d'un récipient de réception dans l'espace de réception (6), dans lequel un axe longitudinal (7) s'étend entre l'extrémité distale (3) et l'extrémité proximale (4),
- un ensemble d'aiguille (9) constitué en tant que canule (10), laquelle canule (10) dépasse dans l'espace de réception (6) par son extrémité de canule (11) proximale en partant de la paroi frontale (5), dans lequel la canule (10) est couplée à la partie de corps principale (2),
- une enveloppe (12) de forme tubulaire, élastiquement déformable ainsi que perforable, qui comporte une extrémité d'enveloppe (13) distale ouverte ainsi qu'une extrémité d'enveloppe (14) proximale fermée et qui, par son extrémité d'enveloppe (14) proximale fermée recouvre l'extrémité de canule (11) proximale,
- et avec un dispositif de retenue (8) avec lequel l'enveloppe (12) est retenue dans la direction axiale, dans lequel le dispositif de retenue (8) pour l'enveloppe (12) est constitué ou disposé directement sur la paroi frontale (5), et en ce que le dispositif de retenue (8) comprend au moins un élément de retenue (16) pour l'enveloppe (12) constitué sur la paroi frontale (5) ou disposé sur la paroi frontale (5), dans lequel l'au moins un élément de retenue (16) est disposé sur le côté de la paroi frontale (5) tourné vers l'espace de réception (6) et comporte un bras de retenue (18) dépassant de la paroi frontale (5), et en ce que l'au moins un élément de retenue (16) est, côté extérieur, adjacent à l'enveloppe (12) dans une zone de contact,
**caractérisé en ce que**
- la canule (10) est raccordée directement à la paroi frontale (5) de la partie de corps principale (2), ou **en ce que** la canule (10) est constituée sur la paroi frontale (5) en tant que partie intégrante de la partie de corps principale (2),
- l'au moins un élément de retenue (16) comporte un bec de retenue (19) disposé sur le bras de retenue (18) ainsi que dépassant en direction de l'axe longitudinal (7),
- un passage (20) traversant la paroi frontale (5) est constitué dans la paroi frontale (5) dans un tronçon de projection, couché vu dans la direction axiale, de l'au moins un élément de retenue (16) près de cet élément dans la direction radiale en direction de l'axe longitudinal (7), et une section transversale du passage (20) correspond dans la direction axiale au moins à une surface de projection du bec de retenue (19).

2. Dispositif de manipulation (1) selon la revendication 1, **caractérisé en ce que** le dispositif de retenue (8) comporte un embout de centrage (15) constitué à peu près de façon cylindrique ou tronconique qui fait partie intégrante de la paroi frontale (5), et l'embout de centrage (15) dépasse dans l'extrémité d'enveloppe (13) distale ouverte de l'enveloppe (12).

3. Dispositif de manipulation (1) selon la revendication 2, **caractérisé en ce que** l'au moins un élément de retenue (16) est disposé à distance radiale de l'embout de centrage (15) avec formation d'un interstice (17).

4. Dispositif de manipulation (1) selon la revendication 3, **caractérisé en ce que** la distance radiale entre l'au moins un élément de retenue (16) et l'embout de centrage (15), quand l'élément de retenue (16) est adjacent à l'enveloppe (12), présente une valeur qui s'inscrit dans une plage de valeurs dont la limite inférieure est égale à 5 % et dont la limite supérieure est égale à 95 % par rapport à l'épaisseur de paroi non déformée de l'enveloppe (12).

5. Dispositif de manipulation (1) selon la revendication 1, **caractérisé en ce qu'**au moins le bec de retenue (19) de l'au moins un élément de retenue (16) est, dans la zone de contact, adjacent à une paroi d'enveloppe de l'enveloppe (12).

6. Dispositif de manipulation (1) selon la revendication 1 ou 5, **caractérisé en ce que** le bec de retenue (19) est disposé à distance de la paroi frontale (5) dans la direction axiale.

7. Dispositif de manipulation (1) selon la revendication 2, **caractérisé en ce que** le passage (20) traversant la paroi frontale (5) est constitué entre le bras de retenue (18) de l'au moins un élément de retenue (16) et l'embout de centrage (15).

8. Dispositif de manipulation (1) selon l'une des revendications précédentes, **caractérisé en ce que** l'enveloppe (12), dans la zone de son extrémité d'enveloppe (13) distale ouverte, comporte une collerette (23) dépassant de l'enveloppe (12) dans la direction radiale.

9. Dispositif de manipulation (1) selon l'une des revendications 5 à 8, **caractérisé en ce que** le bec de retenue (19) de l'au moins un élément de retenue (16) agrippe par l'arrière la collerette (23) de l'enveloppe (12) sur son côté tourné vers l'extrémité proximale (4), constituée de façon ouverte, de la partie de corps principale (2).

10. Dispositif de manipulation (1) selon l'une des revendications précédentes, **caractérisé en ce que** l'au moins un élément de retenue (16) est retenu sur la paroi frontale (5) de façon déplaçable, en particulier pivotante.

11. Dispositif de manipulation (1) selon la revendication 1, **caractérisé en ce que** la canule (10) raccordée directement à la paroi frontale (5) de la partie de corps principale (2) est enfoncée par pression dans un orifice de réception (24) constitué dans la zone de la paroi frontale (5).

12. Dispositif de manipulation (1) selon l'une des revendications précédentes, **caractérisé en ce que** aussi bien l'orifice de réception (24) pour la canule (10) que l'embout de centrage (15) pour l'enveloppe (12) sont disposés respectivement de façon couchée dans l'axe longitudinal (7).

13. Dispositif de manipulation (1) selon la revendication 2, **caractérisé en ce que** la canule (10) est constituée sur l'embout de centrage (15) en tant que partie intégrante de la partie de corps principale (2).

14. Dispositif de manipulation (1) selon l'une des revendications précédentes, **caractérisé en ce qu'**une pièce de raccordement (21) dépassant de la paroi frontale (5) dans la direction axiale est constituée sur la paroi frontale (5) sur le côté éloigné de l'espace de réception (6).

15. Dispositif de manipulation (1) selon la revendication 14, **caractérisé en ce que**, par son extrémité de canule (25) distale, la canule (10) dépasse au moins partiellement dans la pièce de raccordement (21).

16. Dispositif de manipulation (1) selon la revendication 14 ou 15, **caractérisé en ce que**, par son extrémité de canule (25) distale, la canule (10) dépasse de la pièce de raccordement (21) dans la direction axiale et comporte, à son extrémité de canule (25) distale, une extrémité de perforation pour la perforation dans une partie de corps.

17. Dispositif de manipulation (1) selon l'une des revendications 14 à 16, **caractérisé en ce qu'**un adaptateur (30) est retenu de façon positionnée sur la pièce de raccordement (21), et au moins un tronçon d'étanchéité (35, 38) est constitué entre la pièce de raccordement (21) et l'adaptateur (30).

18. Procédé de fabrication d'un dispositif de manipulation (1), en particulier pour la technique médicale, qui comprend les étapes suivantes :
- formation d'une enveloppe (12) de forme tubulaire, élastiquement déformable ainsi que perforable, avec une extrémité d'enveloppe (13) distale ouverte ainsi qu'une extrémité d'enveloppe (14) proximale fermée ;
- formation d'une partie de corps principale (2) creuse avec une extrémité distale (3) fermée au moins partiellement avec une paroi frontale (5) et avec une extrémité proximale (4) ouverte, où un espace de réception (6) est défini par la partie de corps principale (2) ainsi que par la paroi frontale (5), et l'extrémité proximale (4) sert à la réception d'au moins un tronçon partiel d'un récipient de réception dans l'espace de réception (6), dans lequel un axe longitudinal (7) s'étend entre l'extrémité distale (3) et l'extrémité proximale (4) ;
- formation d'un dispositif de retenue (8) pour l'enveloppe (12) directement dans la zone de la paroi frontale (5) sur son côté tourné vers l'espace de réception (6), dans lequel le dispositif de retenue (8) est formé par au moins un élément de retenue (16) constitué sur la paroi frontale (5) ou disposé sur la paroi frontale (5), dans lequel l'au moins un élément de retenue (16) est formé par un bras de retenue (18) dépassant de la paroi frontale (5) ;
- formation d'un bec de retenue (19) sur le bras de retenue (18) de l'au moins un élément de retenue (16), dans lequel le bec de retenue (19) est constitué en faisant saillie en direction de l'axe longitudinal (7) ;
- formation d'un passage (20) traversant la paroi frontale (5), dans lequel le passage (20) est constitué dans un tronçon de projection, couché vu dans la direction axiale, de l'au moins un élément de retenue (16) près de cet élément dans la direction radiale en direction de l'axe longitudinal (7), et une section transversale du passage (20) est constituée dans la direction axiale au moins avec une surface de projection correspondant au bec de retenue (19) ;
- disposition et raccordement d'un ensemble d'aiguille (9) constitué en tant que canule (10) directement avec la paroi frontale (5) de la partie de corps principale (2) ou formation d'un ensemble d'aiguille (9) constitué en tant que canule (10) sur la paroi frontale (5) en tant que partie intégrante de la partie de corps principale (2), de telle sorte que la canule (10) dépasse dans l'espace de réception (6) par son extrémité de canule (11) proximale en partant de la paroi frontale (5) ;
- transfert de l'enveloppe (12) avec son extrémité d'enveloppe (13) distale ouverte dans la zone de l'au moins un élément de retenue (16) constitué sur la paroi frontale (5) ou disposé sur la paroi frontale (5) et conjointement recouvrement de la canule (10) dépassant dans l'espace de réception (6) ;
- réalisation entre l'élément de retenue (16) et l'enveloppe (12) d'une zone de contact dans laquelle l'élément de retenue (16) est, côté extérieur, amené en appui sur l'enveloppe (12).

19. Procédé selon la revendication 18, **caractérisé en ce qu'**un embout de centrage (15) constitué sur la paroi frontale (5) à peu près de façon cylindrique ou tronconique en tant que composant supplémentaire du dispositif de retenue (8) est constitué en tant que partie intégrante de la paroi frontale (5), lequel embout de centrage (15) est constitué en dépassant dans l'extrémité d'enveloppe (13) distale ouverte de l'enveloppe (12).

20. Procédé selon la revendication 19, **caractérisé en ce que** l'au moins un élément de retenue (16) est disposé à distance radiale de l'embout de centrage (15) avec formation d'un interstice (17).

21. Procédé selon la revendication 19, **caractérisé en ce que** le passage (20) traversant la paroi frontale (5) est constitué entre le bras de retenue (18) de l'au moins un élément de retenue (16) et l'embout de centrage (15).

22. Procédé selon l'une des revendications 18 à 21, **caractérisé en ce que** l'enveloppe (12), dans la zone de son extrémité d'enveloppe (13) distale ouverte, comporte une collerette (23) dépassant de l'enveloppe (12) dans la direction radiale.

23. Procédé selon la revendication 20 ou 22, **caractérisé en ce que**, avant la pose de l'enveloppe (12) sur l'embout de centrage (15), l'interstice (17) constitué entre l'au moins un élément de retenue (16) et l'embout de centrage (15) est constitué avec une distance plus grande par rapport à l'épaisseur de paroi de l'enveloppe (12), et après le transfert de l'enveloppe (12) dans l'interstice (17), l'au moins un élément de retenue (16) est déplacé en direction de l'axe longitudinal (7) et donc amené côté extérieur en appui sur l'enveloppe (12) dans la zone de contact.
